# EUROPEAN PATENT APPLICATION

(11) **EP 4 471 146 A1**
(43) Date of publication of application: **04.12.2024**
(21) Application number: 23746358.3
(22) Date of filing: 27.01.2023
(51) Int. Cl.: C12N 15/113

(54) **SMALL RNA DRUG FOR INHIBITING ACTIVITY OF CANCER CELLS**

(30) Priority: 28.01.2022 CN 202210106273
(71) Applicant: Institute of Basic Medical Sciences, Chinese Academy of Medical Sciences, Beijing 100005 (CN)
(72) Inventor: JIANG, Chengyu, Beijing 100005 (CN); SUN, Na, Beijing 100005 (CN); LIN, Yexuan, Beijing 100005 (CN)
(74) Representative: Cabinet Beau de Loménie
(86) International application number: PCT/CN2023/073496
(87) International publication number: WO 2023/143480

(57) **Abstract**

Provided is a small RNA drug for inhibiting the activity of cancer cells. Specifically, provided are an isolated nucleic acid molecule as shown in any one of SEQ ID NO: 1 to SEQ ID NO: 133, or an isolated nucleic acid molecule as shown in a sequence having at least 70%, at least 75%, at least 80%, at least 85%, at least 90%, at least 95%, 96%, 97%, 98, and 99% sequence identity therewith, a vector, a host cell, and a pharmaceutical composition comprising same, and a use thereof in treatment of cancers.

## Description

The present application claims the priority of the Chinese patent application CN202210106273.9 filed on January 28, 2022, and the full text of the above Chinese patent application is cited in the present application.

### FIELD OF THE INVENTION

The present invention relates to the field of nucleic acid therapy, more specifically to a small RNA capable of treating and/or preventing cancer, a composition comprising the same, and a use thereof.

### BACKGROUND OF THE INVENTION

Cancer is a large group of diseases, the other common names for which are tumor and malignant tumor. It is mainly characterized by sustained proliferation, evasion of growth inhibition, evasion of immune clearance, infinite replication, pro-inflammation, activation of infiltration and metastasis, pro-angiogenesis, instability and mutation of genome, resistance to cell death, loss of control of cellular energy metabolism, unlocked phenotypic plasticity, senescent cells, non-mutant epigenetic reprogramming, and polymorphic microbiome. Cancer is the second leading cause of death globally, and the global burden of cancer continues to increase, placing enormous physical, emotional, and financial pressures on individuals, families, communities, and health systems.

Primary bronchogenic carcinoma, or lung cancer for short, is one of the most common malignant tumors worldwide, with an estimated 1.8 million new cases each year, and more than 85% of cases have a poor prognosis within five years of diagnosis. In January 2019, the National Cancer Center released the latest edition of the "Analysis Report on the Epidemic of Malignant Tumors in China", showing that lung cancer is the predominant malignant tumor in China, with 784,000 new cases, and the incidence and mortality rate account for 20.03% and 26.99% of all malignant tumors, respectively, ranking first among all cancers, while the incidence and mortality rate of lung cancer in men are significantly higher than those in women. Lung cancer is classified into non-small cell lung cancer (NSCLC) and small cell lung cancer (SCLC) according to histopathology. Approximately 80% of patients with lung cancer have NSCLC, which originates from lung epithelial cells. NSCLC is further classified into adenocarcinoma, squamous cell carcinoma, and large cell carcinoma, of which the predominant adenocarcinoma accounts for about 40% of NSCLC, and the incidence is still rising, and is the most common type of cancer among women and non-smokers, while squamous carcinoma (SCC) accounts for about 25% of the incidence of NSCLC, and large cell carcinoma accounts for about 15%. SCLC accounts for about 15% of lung cancers and originates from neuroendocrine cells in the bronchi, with a high degree of malignancy. Clinically, in older patients who smoke, squamous cell carcinoma is relatively common, while cases with mixed tissue tumors and large cell carcinoma are rare. As the early symptoms of lung cancer are not obvious, the best treatment period is often missed, and nearly half of the patients have metastases to the brain, adrenal glands, bones and liver at the time of diagnosis, or the lesion is hard to be completely resected, resulting in a five-year survival rate of about 15%. There are many risk factors for lung cancer, among which smoking is one of the most important factors. WHO affirms that tobacco use is one of the main risk factors for death due to lung cancer, while on the other hand, still, 15% of NSCLC patients are non-smokers. Symptoms of NSCLC patients are broadly classified into primary lesion-related symptoms, intrathoracic symptoms, extrapulmonary symptoms, infiltrating and metastatic symptoms, etc. Among the patients, about 50% to 75% of patients have cough as the most common symptom, followed by hemoptysis, chest pain and dyspnea. At present, histological examination is still used clinically to classify the type and stage of the patient's tumor, and to select the treatment method on this basis.

The common treatment methods for lung cancer in clinical guidelines are surgery, radiotherapy and chemotherapy. According to the TNM staging criteria for lung cancer, patients with stage I and II NSCLC and some patients with stage III NSCLC meet the conditions for surgical resection. Surgical resection is not recommended for some patients with stage III A and III B NSCLC. At present, the recommended treatment for stage I disease is concurrent chemoradiotherapy, such as a combination of sequential chemoradiotherapy with platinum as the mainstay and drugs such as paclitaxel, platinum, gemcitabine, etc. For patients with NSCLC, the drugs, most of which are monoclonal antibodies, are selected based on the mutation of the driver gene detected by sequencing technology. With the development of molecular biology and histology techniques, targeted therapies for NSCLC have emerged, making the treatment regimen more individualized and precise. The treatment of lung cancer has entered the era of precise stratified treatment. Targeted therapy is the most typical and valuable embodiment of stratified diagnosis and treatment. According to the latest NSCLC guidelines released by the National Comprehensive Cancer Network (NCCN) in 2019, the mutation-driven genes associated with targeted therapy are: epidermal growth factor receptor (EGFR), anaplastic lymphoma kinase (ALK), c-ros oncogene 1 receptor tyrosine kinase (ROS1), human epidermal growth factor receptor 2 (HER2), mesenchymal to epithelial transition factor (MET), v-raf murine sarcoma viral oncogene homolog B1 (BRAF), kirsten rat sarcoma (KRAS), rearranged during transfection (RET), and neurotrophic tyrosine receptor kinase (NTRK).

Pancreatic cancer is a very lethal malignant tumor. According to the WHO classification, pancreatic malignancies can be classified as epithelial-originated and non-epithelial-originated according to their tissue origins, among which the epithelial-originated ones mainly include ductal adenocarcinoma, acinar cell carcinoma, and neuroendocrine tumor derived from ductal epithelium, acinar cells and neuroendocrine cells, and various mixed tumors. According to the latest epidemiological survey released by CA, there were 495,773 new cases of pancreatic ductal adenocarcinoma and 466,003 new deaths in the world in 2020, ranking 7th in the proportion of deaths, and the number of new cases and deaths was almost the same. It can be seen that pancreatic cancer has a very high mortality rate and a great degree of malignancy, and is known as the king of cancer, and about 90% of pancreatic cancer patients are classified as pancreatic ductal adenocarcinoma. And with the increase in obesity, diabetes and alcohol consumption, it is still showing a steady upward trend. A study on 28 European countries predicts that by 2025, pancreatic cancer will surpass breast cancer and become the third leading cause of cancer death. Pancreatic ductal adenocarcinoma (PDA) is the most common (more than 90%) malignant tumor in pancreatic cancer, with a low 5-year survival rate of about 7.7%, a median survival of less than 6 months, and a 5-year survival of less than 3% once distant metastases occur. In 2018, it caused a total of 458,898 cancer cases and 432,242 deaths worldwide, ranking 14th and 7th in cancer incidence and death, respectively, and showing an upward trend. PDA is characterized by the continuous accumulation of key oncogene mutation (K-Ras, more than 90%), tumor suppressor gene modification (TP53, more than 75%), CDKN2 inactivation (more than 95%), and low or absent expression of Smad4, etc. Another characteristic is that it has a unique tumor microenvironment, comprising a variety of different components including vascular network, extracellular matrix, and a variety of cell types including immune cells, fibroblasts, etc., and extracellular components including growth factors, cytokines, etc.

sRNA derived from traditional Chinese medicine is a new active ingredient of traditional Chinese medicine extracted and discovered in decoction of traditional Chinese medicine in recent years. It is a new interpretation of the efficacy of traditional Chinese medicine, and is a precision medical drug that can target and regulate gene expression. The inventors have a good research foundation in sRNAs derived from traditional Chinese medicine. With the continuous discovery of various targets year by year and the continuous research and development of various targeted drugs, the drug treatment of lung cancer and pancreatic cancer is truly entering the era of precision treatment from traditional chemotherapy. Although lung cancer and pancreatic cancer have long been considered as diseases diagnosed at late stages, if there is a breakthrough in the treatment regimen and the symptoms of patients can be quickly and effectively improved, it would become a treatment choice that scientific researchers, pharmaceutical companies and patients all hope for.

### SUMMARY OF THE INVENTION

The inventors have unexpectedly discovered small RNAs capable of suppressing cancer, and therefore, the present application is filed.

The present application is partly based on the discovery of small RNAs by the inventors. The inventors have discovered that the small RNAs in the present application can inhibit the cellular activity of lung cancer cells. The inventors have discovered that the small RNAs in the present application can inhibit the cellular activity of pancreatic cancer cells.

The inventor screened small RNAs from traditional Chinese medicine that have the function of inhibiting the activity of lung cancer or pancreatic cancer cells. Lung cancer cell models: H460 cells, A549 cells and H23 cells; pancreatic cancer cell models: PANC-1 cells, ASPC-1 cells, HS 766T cells, BXPC-3 cells; multi-spectrum cell models: PC-3 (prostate cancer), MCF-7 (breast cancer), MDA-MB-231 (breast cancer), MKN45 (gastric cancer), HCT116 (colon cancer), AGS (gastric adenocarcinoma), ASPC1 (pancreatic cancer), HS766T (pancreatic cancer), BXPC-3 (pancreatic cancer), A549 (lung cancer). All cells were purchased from the Cell Center of the Chinese Academy of Medical Sciences.

Specifically, the present invention provides the following small RNAs with the activity of suppressing lung cancer cells, the specific sequences of which are as shown in Table 1 below:

**Table 1**

| Name | Sequence | SEQ ID NO: |
|---|---|---|
| LQi-sRNA-3 | UAGUGGUAUGAUUCUCGC | 1 |
| PGY-sRNA-6 | GUUCAGAGUUCUACAGUCCGA | 2 |
| CHu-sRNA-48 | UGCUGUAGUAGGUUGUAUAG | 3 |
| YXC-sRNA-39 | UGCUGUAGUAGUUUGUGCUGU | 4 |
| YXC-sRNA-62 | UCUUCCCAGUGCUCUGAAUGU | 5 |
| HQi-sRNA-2 | AUGGUUCGAUUCCGGAGAGGG | 6 |
| YXC-sRNA-49 | AGGGUUCGAGUGUGAGCAUGC | 7 |
| BZL-sRNA-15 | GGGGGCGUAGCUCAGAUGGU | 8 |
| BZL-sRNA-25 | UGGAAACGGCUGCUAAUACC | 9 |
| BZL-sRNA-32 | AGCUGGAAACGGCUGCUAAUACC | 10 |
| BZL-sRNA-34 | GAGCCCCGUCGUGCCCGGACC | 11 |
| CHu-sRNA-1 | ACAACUUUCAGCAACGGA | 12 |
| BHSC-sRNA-15 | UACCUGGUUGAUUCUGCCA | 13 |
| BHSC-sRNA-17 | UGUGGGCACUCAAAGUGAC | 14 |
| BHSC-sRNA-18 | UGUGGGCACUCAAAGUGA | 15 |
| BHSC-sRNA-22 | UGUGGGUGCUUGUGAGUACGGACU | 16 |
| BHSC-sRNA-24 | UACCUGGUUGAUCCUGCCA | 17 |
| BHSC-sRNA-42 | UAUCUGGUUGAUUCUGCC | 18 |
| MDM-sRNA-1 | UCCGUUGUCGUCCAGCGGUUAGGAUAUCUGG | 19 |
| MDM-sRNA-4 | GCGUCUGUAGUCCAACGGUUAGGAUAAUUGC | 20 |
| MDM-sRNA-7 | GCGUCUGUAGUCCAACGGUUAGGAUA | 21 |
| MDM-sRNA-11 | AGGGCACGUCUGCCUGGGUGUCACGU | 22 |
| MDM-sRNA-12 | CUGGGAAGUCCUCGUGUUGCACCCUC | 23 |
| MDM-sRNA-22 | AUUCGGUCGGGAACUCAAAGGAGACUGCC | 24 |
| MDM-sRNA-27 | UCCAUUGUCGUCUAGUCCGGUUUAGGAUA | 25 |
| MDM-sRNA-28 | GGGGAUGUAGCUCAGAUGG | 26 |
| MDM-sRNA-33 | GAAACGACUCUCGGCAACGGAUAUC | 27 |
| MDM-sRNA-38 | CUUGAGUUCGACUCUCAACGAGAGCACCA | 28 |
| MDM-sRNA-39 | GGGAUUGUAGUUCAAUUGGUCAGAGCACC | 29 |
| MDM-sRNA-41 | UGAAACGACUCUCGGCAACGGAUAUC | 30 |
| MDM-sRNA-163 | UGACUCUAGUCCGACUUUGUGAAAUGACU | 31 |

In another aspect, the present invention provides small RNAs with multi-spectrum anti-cancer function, which can be used to suppress prostate cancer, breast cancer, gastric cancer, colon cancer, pancreatic cancer and lung cancer. The cancer cell lines used in the present invention are: PC-3 (prostate cancer), MCF-7 (breast cancer), MDA-MB-231 (breast cancer), MKN45 (gastric cancer), HCT116 (colon cancer), AGS (gastric adenocarcinoma), ASPC1 (pancreatic cancer), HS766T (pancreatic cancer), A549 (lung cancer). The specific sequences of the small RNAs are shown in Table 2 below:

**Table 2**

| Name | Sequence | SEQ ID NO: |
|---|---|---|
| MDM-sRNA-1 | UCCGUUGUCGUCCAGCGGUUAGGAUAUCUGG | 19 |
| MDM-sRNA-4 | GCGUCUGUAGUCCAACGGUUAGGAUAAUUGC | 20 |
| MDM-sRNA-7 | GCGUCUGUAGUCCAACGGUUAGGAUA | 21 |
| MDM-sRNA-11 | AGGGCACGUCUGCCUGGGUGUCACGU | 22 |
| MDM-sRNA-12 | CUGGGAAGUCCUCGUGUUGCACCCUC | 23 |
| MDM-sRNA-22 | AUUCGGUCGGGAACUCAAAGGAGACUGCC | 24 |
| MDM-sRNA-27 | UCCAUUGUCGUCUAGUCCGGUUUAGGAUA | 25 |
| MDM-sRNA-28 | GGGGAUGUAGCUCAGAUGG | 26 |
| MDM-sRNA-33 | GAAACGACUCUCGGCAACGGAUAUC | 27 |
| MDM-sRNA-38 | CUUGAGUUCGACUCUCAACGAGAGCACCA | 28 |
| MDM-sRNA-39 | GGGAUUGUAGUUCAAUUGGUCAGAGCACC | 29 |
| MDM-sRNA-41 | UGAAACGACUCUCGGCAACGGAUAUC | 30 |
| MDM-sRNA-163 | UGACUCUAGUCCGACUUUGUGAAAUGACU | 31 |

On the other hand, the present invention provides small RNAs with anti-pancreatic cancer function, which functionally inhibits the activity of pancreatic cancer cells. The cancer cell lines used in the present invention are: HS 766T (pancreatic cancer), PANC-1 (pancreatic cancer), BXPC-3 (pancreatic cancer); the specific sequences of the small RNAs are as shown in Table 3 below:

**Table 3**

| Name | Sequence | SEQ ID NO: |
|---|---|---|
| Hqi-sRNA-2 | AUGGUUCGAUUCCGGAGAGGG | 6 |
| BHSC-sRNA-15 | UACCUGGUUGAUUCUGCCA | 13 |
| MDM-sRNA-1 | UCCGUUGUCGUCCAGCGGUUAGGAUAUCUGG | 19 |
| MDM-sRNA-4 | GCGUCUGUAGUCCAACGGUUAGGAUAAUUGC | 20 |
| MDM-sRNA-7 | GCGUCUGUAGUCCAACGGUUAGGAUA | 21 |
| MDM-sRNA-22 | AUUCGGUCGGGAACUCAAAGGAGACUGCC | 24 |
| MDM-sRNA-27 | UCCAUUGUCGUCUAGUCCGGUUUAGGAUA | 25 |
| MDM-sRNA-33 | GAAACGACUCUCGGCAACGGAUAUC | 27 |
| MDM-sRNA-38 | CUUGAGUUCGACUCUCAACGAGAGCACCA | 28 |
| MDM-sRNA-39 | GGGAUUGUAGUUCAAUUGGUCAGAGCACC | 29 |
| MDM-sRNA-41 | UGAAACGACUCUCGGCAACGGAUAUC | 30 |
| MDM-sRNA-163 | UGACUCUAGUCCGACUUUGUGAAAUGACU | 31 |
| LGT-sRNA-8 | GGUGGCUGUAGUUUAGUGGUAAGAAUUCCACG UU | 32 |
| LGT-sRNA-10 | CCGACUGUUUAAUUAAAACA | 33 |
| LGT-sRNA-14 | UCCGUUGUCGUCCAGCGGUUAGGAUAUCUGGC | 34 |
| LGT-sRNA-16 | UCCUCUGUAGUUCAGUCGGUAGAACGGC | 35 |
| LGT-sRNA-17 | UCCGACUGUUUAAUUAAAACA | 36 |
| LGT-sRNA-20 | UCCGACUUUGUGAAAUGA | 37 |
| LGT-sRNA-21 | UCCGACUGUUUAAUUAAAACAAAGCA | 38 |
| LGT-sRNA-22 | AGGCUUGUAGCUCAGGUGGUUAGAGCGCA | 39 |
| LGT-sRNA-23 | UCCGUUGUCGUCCAGCGGUUAGGAUAUCUG | 40 |
| LGT-sRNA-28 | CUCUAGUCCGACUUUGUGAAA | 41 |
| LGT-sRNA-29 | CGUCGUCGCGGCGAUCGUG | 42 |
| LGT-sRNA-30 | UGAACUAAUUCAGACUGUG | 43 |
| LGT-sRNA-34 | CUCUAGUCCGACUUUGUGAA | 44 |
| LGT-sRNA-35 | CAAAACGACUCUCGGCAACGGA | 45 |
| LGT-sRNA-38 | GUAAGGAUUGACAGACUG | 46 |
| LGT-sRNA-39 | CGACUCUCGGCAACGGAUA | 47 |
| LGT-sRNA-43 | GUGCUCUGAAUGUCAAAGUGA | 48 |
| LGT-sRNA-46 | AAAACGACUCUCGGCAACGGAUA | 49 |
| TYSW10 | CCCUAUCAACUUUCGAUGGUAGG | 50 |
| TYSW29 | AUAUCUCGGCUCACGCAUCG | 51 |
| TYSW30 | CGUUGCUUUUUGAUCCUUCG | 52 |
| TYSW31 | CUGCCGGCGGACUGCUCGAGC | 53 |
| TYSW60 | UUCACGUCGGGUUCACCA | 54 |
| TYSW104 | UUGUCGUCCAGCGGUUAGGAUA | 55 |
| TYSW105 | CCGGAGACGUCGGCGGGGGCCUCGGGA | 56 |
| TYSW106 | UGAUUCAUGAUAACUCGUCGGA | 57 |
| TYSW107 | ACAGACUGAGAGCUCUUUC | 58 |
| TYSW108 | UCCUUAGUUCGAUCCUGAGUGCGAGCUC | 59 |
| TYSW109 | UACUGACCGGGGUUCGUUUCCCCGGA | 60 |
| TYSW110 | UGAUAACUCGUCGGAUCGCA | 61 |
| TYSW112 | CCCGCCGCCUGUUUGCCGAC | 62 |
| TYSW113 | UGGUCCCUGCGGAUGCUCA | 63 |
| TYSW115 | CUUCACUGGCUGUGGGGGGAACC | 64 |
| TYSW 116 | GUGCUUGAAAUUGUCGGGAGGGA | 65 |
| TYSW117 | AUGAACAAAUUCAGACUGUG | 66 |
| TYSW118 | GGCCGGGGGACGGACUGGGA | 67 |
| TYSW 119 | ACCCGUCGGCUGUCGGCGA | 68 |
| TYSW120 | CACACCGCCCGUCGCUCCU | 69 |
| TYSW121 | UAAUAGAAUAGGACGUGUG | 70 |
| TYSW122 | CAGUCAACUCAGAACUGGUACGGA | 71 |
| TYSW123 | GAACCGUUGAUUCGCACA | 72 |
| TYSW124 | CCCUCCGAAGUUUCCCUCAGG | 73 |
| TYSW126 | CGACUCUCGACAACGGAUA | 74 |
| TYSW129 | CCGACCUUAGCUCAGUUGGU | 75 |
| TYSW131 | UAACUCGUCGGAUCGCACG | 76 |
| TYSW132 | CCGGCUGGGGGACGGACUGGGA | 77 |
| TYSW133 | GGUGGCUGUAGUUUAGUGGUGAGA | 78 |
| TYSW134 | GUCCGAUCAACUGUAGUU | 79 |
| TYSW135 | CCACCGACCUAAGUUCCUUGGAACA | 80 |
| TYSW136 | UGCAAACUCCGAAUACCUACA | 81 |
| TYSW137 | GCGAUUCUGACGUGCAAAUCGAUCGUCA | 82 |
| TYSW138 | CGAAACUCAGGUGCUGCAAUC | 83 |
| TYSW139 | UCCCGAACUUGGUGGUUAAACUCUA | 84 |
| TYSW140 | CUUAUUCCGUGAAUCGGA | 85 |
| TYSW141 | UCAACUUUCGAUGGUAGGA | 86 |
| TYSW143 | UUCCGUCCAAGGCUAAAUACUG | 87 |
| TYSW162 | GCCGGCCGGGGGACGGACUG | 88 |
| TYSW163 | AUUUUGUUGGUUUCUAGGACC | 89 |
| TYSW164 | CCCGGUGGCGGACGCUCUUGGCA | 90 |
| TYSW165 | AAGGAUUGGCUCUGAGGGCUG | 91 |
| TYSW166 | CGGGUCGUGAACUUCUUUUCCCGGA | 92 |
| TYSW167 | UUUCGGUCUUAUUACGUU | 93 |
| TYSW168 | CGGAUAUCUCGGCUCACGCA | 94 |
| TYSW169 | ACUUGAGAGGUGUAGGAUA | 95 |
| TYSW170 | CCGACCUUAGCUCAGUUGGC | 96 |
| TYSW171 | AUUAGUGGAACGCUCUGGAAAGUGC | 97 |
| TYSW172 | UCGAUUCCGGGCUUGCGCACCA | 98 |
| TYSW173 | AGCUCGUUUGAUUCUGAUUUCC | 99 |
| TYSW175 | AUCCACGGCCAUAGGACUCUG | 100 |
| TYSW176 | GGGUGAAGGACUUGCUCCGUA | 101 |
| TYSW177 | UGACGAGUGUUCUUUUAGA | 102 |
| TYSW178 | UCUCUGAAUGGGGGAACCCACUC | 103 |
| TYSW179 | CGGACUGCUCGAGCUGCCUCG | 104 |
| TYSW260 | CGUUUGGAAUUCUGGAGACG | 105 |
| TYSW460 | CCGUCGCUAGCUUGGUUGAA | 106 |
| TYSW461 | UCCGAUCAACUGUAGUUA | 107 |
| TYSW462 | CUUUGAAUUUGCUUGUGUAGGAUA | 108 |
| TYSW463 | GUUCGAUUCUCGGCGAGAGAGCCA | 109 |
| TYSW464 | UCUUGACGACCAUAGAGCAUUG | 110 |
| TYSW465 | UUGGUCUUGUAAUUGGAAUG | 111 |
| TYSW475 | CGACUCAGAACUGGUACG | 112 |
| TYSW476 | AGGGAAAGAUGAAAAGGACU | 113 |
| TYSW478 | AACCUUAACCUAUUCUCAAAC | 114 |
| TYSW480 | UGUGUCGCUUCGAUUCGU | 115 |
| TYSW483 | CGUCUGCCUGGGUGUCACGU | 116 |
| TYSW484 | GUCUGCCUGGGCGUCACGU | 117 |
| TYSW494 | CUGGUCGGGUCGUGCCUCC | 118 |
| TYSW497 | CCCCGGGCGCGAGCCCGGGCGGA | 119 |
| TYSW502 | UCUCCGAUCGUAUAGUGGCUAGUA | 120 |
| TYSW538 | UCCCGAACCCGCCGGCUGUCG | 121 |
| TYSW746 | CGCGGGCUCUGCCCGUUGCUGCGA | 122 |
| TYSW751 | GUUUGUUUGAUGGUAUCUACUACU | 123 |
| TYSW753 | CGGACUGCUCGAGCUGCUUCC | 124 |
| TYSW754 | CAAGUGGAAGUGCAGUGAUGUAUGCAG | 125 |
| TYSW755 | GUCUUUGGGUUCCGGGGGG | 126 |
| TYSW761 | GUCCCGAGUUCGUUUCUCGGA | 127 |
| TYSW767 | CUGACUCAGAACUGGUACG | 128 |
| TYSW768 | CAUGUCUGCCUGGGUGUCACGC | 129 |
| TYSW770 | UCGUGGGUUCGGGUCCCACGGA | 130 |
| TYSW781 | UCCAUUCUCAAACUUUAAAUA | 131 |
| TYSW785 | CCGAUGUCGUCCAGCGGUUAGGAUA | 132 |
| TYSW791 | UCGCGUGCCGGCCGGGGGA | 133 |

The 133 small RNAs in Table 1 were clustered according to the similarity of nucleic acid sequences using the sequence clustering software CD-HIT. For a group of small RNAs in the same cluster, the small RNA with the largest sequence length in the group was the reference sequence, and the continuous nucleotides that were exactly the same among the small RNAs in the group were the core sequence.

The sequence similarity of a small RNA was that, dividing the length of the nucleic acids of the small RNA consistent with the reference sequence of the group by the total length of the nucleic acid sequence of the small RNA. The sequence similarities of other small RNAs in the same group with respect to the reference sequences of the group were set to greater than or equal to 70% (program parameter "-C 0.7"). For the length of the nucleic acids consistent with the reference sequence of the group, the threshold for inclusion for calculation was: the length of the continuous nucleotides of the small RNA that were exactly the same as the aligned reference sequence of the group was not less than 5 (program parameter "-n 5").

The 133 small RNAs in Tables 1, 2 and 3 could be clustered into 78 groups by CD-HIT when the above conditions were met, and each group had at least two sequences. The groups, the reference sequence of each group, the small RNAs comprised therein, and the shared core sequences within the group were as shown in Table 4.

**Table 4: sRNA grouping and clustering and targets of action**

| Group | Reference sequence | Core sequence | Small RNAs within the same group |
|---|---|---|---|
| Cluster 1 | LQi-sRNA-3 | UAGUGGU | LQi-sRNA-3, TYSW133 |
| Cluster 2 | LGT-sRNA-14 | UAGGAUA | MDM-sRNA-1, MDM-sRNA-4, MDM-sRNA-7, LGT-sRNA-14, MDM-sRNA-27, LGT-sRNA-14, LGT-sRNA-23, TYSW104, TYSW169, TYSW785, |
| Cluster 3 | MDM-sRNA-22 | AACUCA | TYSW138, MDM-sRNA-22 |
| Cluster 4 | MDM-sRNA-38 | GUUCGA | HQi-sRNA-2, MDM-sRNA-38, YXC-sRNA-49, TYSW463 |
| Cluster 5 | MDM-sRNA-39 | UUGUAG | LGT-sRNA-22, MDM-sRNA-39 |
| Cluster 6 | MDM-sRNA-163 | UUUGUG | YXC-sRNA-39, LGT-sRNA-20, LGT-sRNA-28, LGT-sRNA-34, MDM-sRNA-163 |
| Cluster 7 | LGT-sRNA-16 | UUCAG | CHu-sRNA-1, LGT-sRNA-16 |
| Cluster 8 | TYSW108 | GAUCCUG | BHSC-sRNA-24, TYSW108 |
| Cluster 9 | TYSW137 | UCGAU | TYSW480, TYSW137 |
| Cluster 10 | TYSW105 | CGGGGG | TYSW791, TYSW105 |
| Cluster 11 | TYSW754 | - | TYSW754 |
| Cluster 12 | MDM-sRNA-11 | GUCUGCCUGGG (SEQ ID NO: 134) | TYSW483, TYSW484, TYSW768, MDM-sRNA-11 |
| Cluster 13 | MDM-sRNA-12 | - | MDM-sRNA-12 |
| Cluster 14 | MDM-sRNA-41 | CGACUCUCG | MDM-sRNA-33, MDM-sRNA-41, LGT-sRNA-35, LGT-sRNA-39, LGT-sRNA-46, TYSW126 |
| Cluster 15 | LGT-sRNA-21 | CCGACUGUUUAAUUAAAACA (SEQ ID NO: 133) | LGT-sRNA-10, LGT-sRNA-17, LGT-sRNA-21, TYSW461 |
| Cluster 16 | TYSW109 | GUUCG | TYSW109, TYSW761, TYSW770 |
| Cluster 17 | TYSW135 | CCGACCUUAGCUCAGUUGG (SEQ ID NO: 135) | TYSW129, TYSW135, TYSW170 |
| Cluster 18 | TYSW139 | - | TYSW139 |
| Cluster 19 | TYSW166 | - | TYSW166 |
| Cluster 20 | TYSW171 | AAAGUG | BHSC-sRNA-17, BHSC-sRNA-18, TYSW171 |
| Cluster 21 | BHSC-sRNA-22 | - | BHSC-sRNA-22 |
| Cluster 22 | TYSW122 | ACUCAGAACUGGUACG (SEQ ID NO: 136) | TYSW122, TYSW475, TYSW767 |
| Cluster 23 | TYSW462 | - | TYSW462 |
| Cluster 24 | TYSW502 | - | TYSW502 |
| Cluster 25 | TYSW746 | - | TYSW746 |
| Cluster 26 | TYSW751 | - | TYSW751 |
| Cluster 27 | BZL-sRNA-32 | UGGAAACGGCUGCUAAUACC (SEQ ID NO: 9) | BZL-sRNA-25, BZL-sRNA-32 |
| Cluster 28 | TYSW10 | UCAACUUUCGAUGGUAGG (SEQ ID NO: 137) | TYSW10, TYSW141 |
| Cluster 29 | TYSW115 | - | TYSW115 |
| Cluster 30 | TYSW116 | - | TYSW116 |
| Cluster 31 | TYSW164 | GGCGGAC | TYSW164, TYSW31 |
| Cluster 32 | TYSW178 | - | TYSW178 |
| Cluster 33 | TYSW497 | - | TYSW497 |
| Cluster 34 | TYSW106 | UGAUAACUCGUCGGA (SEQ ID NO: 138) | TYSW106, TYSW110 |
| Cluster 35 | TYSW132 | GGGGGACGGACUG (SEQ ID NO: 139) | TYSW118, TYSW132, TYSW162 |
| Cluster 36 | TYSW172 | - | TYSW172 |
| Cluster 37 | TYSW173 | UUGAUUCUG | BHSC-sRNA-15, BHSC-sRNA-42, TYSW173 |
| Cluster 38 | TYSW464 | - | TYSW464 |
| Cluster 39 | PGY-sRNA-6 | - | PGY-sRNA-6 |
| Cluster 40 | YXC-sRNA-62 | - | YXC-sRNA-62 |
| Cluster 41 | BZL-sRNA-34 | - | BZL-sRNA-34 |
| Cluster 42 | LGT-sRNA-43 | - | LGT-sRNA-43 |
| Cluster 43 | TYSW124 | - | TYSW124 |
| Cluster 44 | TYSW136 | - | TYSW136 |
| Cluster 45 | TYSW163 | - | TYSW163 |
| Cluster 46 | TYSW165 | AAGGAUUG | LGT-sRNA-38, TYSW165 |
| Cluster 47 | TYSW175 | - | TYSW175 |
| Cluster 48 | TYSW176 | - | TYSW176 |
| Cluster 49 | TYSW478 | - | TYSW478 |
| Cluster 50 | TYSW538 | CGGCUGUCG | TYSW119, TYSW538 |
| Cluster 51 | TYSW753 | GACUG | TYSW107, TYSW753 |
| Cluster 52 | TYSW781 | - | TYSW781 |
| Cluster 53 | CHu-sRNA-48 | - | CHu-sRNA-48 |
| Cluster 54 | BZL-sRNA-15 | GUAGCUCAGAUGG (SEQ ID NO: 140) | MDM-sRNA-28, BZL-sRNA-15 |
| Cluster 55 | TYSW29 | AUAUCUCGGCUCACGCA (SEQ ID NO: 141) | TYSW29, TYSW168 |
| Cluster 56 | TYSW30 | - | TYSW30 |
| Cluster 57 | TYSW112 | - | TYSW112 |
| Cluster 58 | LGT-sRNA-30 | AAUUCAGACUGUG (SEQ ID NO: 142) | LGT-sRNA-30, TYSW117 |
| Cluster 59 | TYSW460 | - | TYSW460 |
| Cluster 60 | TYSW465 | - | TYSW465 |
| Cluster 61 | TYSW476 | - | TYSW476, |
| Cluster 62 | LGT-sRNA-29 | - | LGT-sRNA-29 |
| Cluster 63 | TYSW113 | - | TYSW113 |
| Cluster 64 | TYSW120 | - | TYSW120 |
| Cluster 65 | TYSW121 | - | TYSW121 |
| Cluster 66 | TYSW131 | UCGCAC | TYSW123, TYSW131 |
| Cluster 67 | TYSW177 | - | TYSW177 |
| Cluster 68 | TYSW494 | - | TYSW494 |
| Cluster 69 | TYSW755 | - | TYSW755 |
| Cluster 70 | TYSW167 | - | TYSW167 |
| Cluster 71 | TYSW143 | - | TYSW143 |
| Cluster 72 | TYSW179 | - | TYSW179 |
| Cluster 73 | TYSW260 | - | TYSW260 |
| Cluster 74 | TYSW140 | - | TYSW140 |
| Cluster 75 | TYSW134 | - | TYSW134 |
| Cluster 76 | TYSW60 | - | TYSW60 |
| Cluster 77 | TYSW461 | - | TYSW461 |
| Cluster 78 | TYSW135 | - | TYSW135 |

Therefore, in one aspect of the present invention, provided is an isolated nucleic acid molecule comprising or consisting of the following sequence:
A) a nucleotide sequence as shown in any one of SEQ ID NO: 1 to SEQ ID NO: 133;
B) a sequence having at least 70%, at least 75%, at least 80%, at least 85%, at least 90%, at least 95%, 96%, 97%, 98%, 99% sequence identity with a nucleotide sequence as shown in any one of SEQ ID NO: 1 to SEQ ID NO: 133;
C) a sequence having at least 10, preferablyat least 9, at least 8, at least 7, at least 6, at least 5, at least 4, at least 3, at least 2, at least 1 nucleotide substitutions, deletions, or additions compared with a nucleotide sequence as shown in any one of SEQ ID NO: 1 to SEQ ID NO: 133,
D) a sequence capable of hybridizing to a nucleotide sequence as shown in any one of SEQ ID NO: 1 to SEQ ID NO: 133 under stringent conditions, or
F) a nucleotide sequence comprising any one of the core sequences listed in Table 4.

In preferred embodiments, the isolated nucleic acid molecule according to the present invention is a RNA molecule or a DNA molecule, preferably a small RNA molecule, preferably a small RNA molecule with a length of 18-36 nucleotides, preferably a small RNA molecule with a length of 18, 19, 20, 21, 22, 23, 24, 25, 26, 27, 28, 29, 30, 31, 32, 33, 34, 35 or 36 nucleotides.

In embodiments of the present invention, the present invention provides an isolated small RNA molecule comprising or consisting of the following sequence:
A) a nucleotide sequence as shown in any one of SEQ ID NO: 1 to SEQ ID NO: 133;
B) a sequence having at least 70%, at least 75%, at least 80%, at least 85%, at least 90%, at least 95%, 96%, 97%, 98%, 99% sequence identity with a nucleotide sequence as shown in any one of SEQ ID NO: 1 to SEQ ID NO: 133;
C) a sequence having up to 10 nucleotide substitutions, deletions, or additions compared with a nucleotide sequence as shown in any one of SEQ ID NO: 1 to SEQ ID NO: 133, preferably a sequence having 9, 8, 7, 6, 5, 4, 3, 2, 1 nucleotide substitution, deletion, or addition,
D) a sequence capable of hybridizing to a nucleotide sequence as shown in any one of SEQ ID NO: 1 to SEQ ID NO: 133 under stringent conditions, or
E) a nucleotide sequence comprising any one of the core sequences listed in Table 4.

In one embodiment of the present invention, the present invention provides an isolated small RNA molecule comprising or consisting of the following sequence:
A) a nucleotide sequence as shown in any one of SEQ ID NO: 6, 7, 28 or 109;
B) a sequence having at least 70%, at least 75%, at least 80%, at least 85%, at least 90%, at least 95%, 96%, 97%, 98%, 99% sequence identity with a nucleotide sequence as shown in any one of SEQ ID NO: 6, 7, 28 or 109, the sequence targets cyclooxygenase-2 and comprises a core sequence GUUCGA; or
C) a nucleotide sequence as shown in SEQ ID NO: 39 or 29;
D) a sequence having at least 70%, at least 75%, at least 80%, at least 85%, at least 90%, at least 95%, 96%, 97%, 98%, 99% sequence identity with a nucleotide sequence as shown in SEQ ID NO: 39 or 29, and the sequence comprises a core sequence UUGUAG; or
E) a nucleotide sequence as shown in SEQ ID NO: 31, 4, 37, 41 or 44;
F) a sequence having at least 70%, at least 75%, at least 80%, at least 85%, at least 90%, at least 95%, 96%, 97%, 98%, 99% sequence identity with a nucleotide sequence as shown in SEQ ID NO: 31, 4, 37, 41 or 44, and the sequence comprises a core sequence UUUGUG.

In embodiments of the present invention, the small RNA of the present invention can be single-stranded or double-stranded.

The double-stranded small RNA molecule of the present invention comprises a sense strand and an antisense strand, wherein the sense strand comprises or consists of the following sequence:
A) a nucleotide sequence as shown in any one of SEQ ID NO: 1 to SEQ ID NO: 133;
B) a sequence having at least 70%, at least 75%, at least 80%, at least 85%, at least 90%, at least 95%, 96%, 97%, 98% or 99% sequence identity with a nucleotide sequence as shown in any one of SEQ ID NO: 1 to SEQ ID NO: 133;
C) a sequence having up to 10 nucleotide substitutions, deletions, or additions compared with a nucleotide sequence as shown in any one of SEQ ID NO: 1 to SEQ ID NO: 133, preferably a sequence having 9, 8, 7, 6, 5, 4, 3, 2, 1 nucleotide substitution, deletion, or addition,
D) a sequence capable of hybridizing to a nucleotide sequence as shown in any one of SEQ ID NO: 1 to SEQ ID NO: 133 under stringent conditions, or
E) a nucleotide sequence comprising any one of the core sequences listed in Table 4.

In embodiments of the present invention, the small RNA molecule provided by the present invention may be a single-stranded or double-stranded small RNA molecule.

In embodiments of the present invention, the small RNA molecule provided by the present invention may be isolated from nature, or may be obtained by artificial synthesis.

In embodiments of the present invention, the double-stranded small RNA molecule according to the present invention comprises a sense strand and an antisense strand, wherein the sense strand comprises or consists of the following sequence:
A) a nucleotide sequence as shown in any one of SEQ ID NO: 1 to SEQ ID NO: 133;
B) a sequence having at least 70%, at least 75%, at least 80%, at least 85%, at least 90%, at least 95%, 96%, 97%, 98%, 99% sequence identity with a nucleotide sequence as shown in any one of SEQ ID NO: 1 to SEQ ID NO: 133;
C) a nucleotide sequence having up to 10 nucleotide substitutions, deletions, or additions compared with a nucleotide sequence as shown in any one of SEQ ID NO: 1 to SEQ ID NO: 133, preferably a nucleotide sequence having 9, 8, 7, 6, 5, 4, 3, 2, 1 nucleotide substitution, deletion, or addition compared with a nucleotide sequence as shown in any one of SEQ ID NO: 1 to SEQ ID NO: 133,
D) a nucleotide sequence capable of hybridizing to a nucleotide sequence as shown in any one of SEQ ID NO: 1 to SEQ ID NO: 133 under stringent conditions, or
E) a nucleotide sequence comprising any one of the core sequences listed in Table 4; and
   the antisense strand comprises a sequence complementary to the sense strand.

In another aspect, the present invention provides a precursor miRNA, which may be processed within the host into the isolated small RNA molecule according to the present invention.

In another aspect, the present invention provides a polynucleotide, which may be transcribed by the host to form the precursor miRNA according to the present invention.

In another aspect, the present invention provides an expression vector comprising the nucleic acid molecule, the isolated small RNA molecule, the precursor miRNA, and/or the polynucleotide according to the present invention.

In another aspect, the present invention provides a host cell transfected with the expression vector of the present invention.

In another aspect, the present invention provides a pharmaceutical composition comprising a therapeutically effective amount of the nucleic acid molecule, the small RNA molecule, the precursor miRNA, the polynucleotide, the expression vector or the host cell according to the present invention, as well as one or more pharmaceutically acceptable adjuvants, excipients and/or stabilizers.

Preferably, the pharmaceutical composition according to the present invention may be used for administration via oral, intramuscular, intravenous, subcutaneous, percutaneous, intraarterial, intraperitoneal, intrapulmonary, intracerebrospinal, intraarticular, intrasynovial, intrathecal, intraventrical, and/or inhalation routes, preferably, the composition according to the present invention is administered orally.

Preferably, in the pharmaceutical composition of the present invention, the content of the nucleic acid molecule, the small RNA molecule, the precursor miRNA, the polynucleotide, the expression vector or the host cell is 0.1-1000 µM, preferably 3.0 µM-300 µM, preferably 0.3 µM, 0.6 µM, 0.9 µM, 1.0 µM, 3.0 µM, 6.0 µM, 9.0 µM, 10.0 µM, 13.0 µM, 16.0 µM, 19.0 µM, 20.0 µM, 23.0 µM, 26.0 µM, 29.0 µM, 30.0 µM, 33.0 µM, 36.0 µM, 39.0 µM, 40.0 µM, 43.0 µM, 46.0 µM, 49.0 µM, 50.0 µM, 53.0 µM, 56.0 µM, 59.0 µM, 60.0 µM, 63.0 µM, 66.0 µM, 69.0 µM, 70.0 µM, 73.0 µM, 76.0 µM, 79.0 µM, 80.0 µM, 83.0 µM, 86.0 µM, 89.0 µM, 90.0 µM, 100 µM, 130 µM, 160 µM, 190 µM, 200 µM, 250 µM, 300 µM, 350 µM, 400 µM, 450 µM, 500 µM, 550 µM, 600 µM, 650 µM, 700 µM, 750 µM, 800 µM, 850 µM, 900 µM, 950 µM, 1000 µM, or any range between these point values.

In another aspect, the present invention provides use of the nucleic acid molecule, the small RNA molecule, the precursor miRNA, the polynucleotide, the expression vector, the host cell or the pharmaceutical composition according to the present invention in the preparation of a medicament for treating cancer; preferably, the cancer is selected from the group consisting of lung cancer, preferably squamous cell carcinoma, small cell lung cancer, non-small cell lung cancer; glioma; digestive system tumor, preferably gastric cancer, colon cancer, rectal cancer, liver cancer, pancreatic cancer; kidney cancer; ovarian cancer; uterine cancer; endometrial cancer; prostate cancer; thyroid cancer; neuroblastoma; brain cancer; glioblastoma multiforme; cervical cancer; bladder cancer; breast cancer; head and neck cancer; rhabdomyosarcoma; Ewing's sarcoma; osteosarcoma; soft tissue sarcoma; nasal NK/T-cell lymphoma; myeloma; melanoma; leukemia, preferably acute lymphoblastic leukemia, acute myelogenous leukemia, acute myeloid leukemia, chronic lymphocytic leukemia, chronic myelogenous leukemia, chronic myeloid leukemia.

Preferably, the nucleic acid molecule, the small RNA molecule, the precursor miRNA, the polynucleotide, the expression vector, the host cell or the pharmaceutical composition according to the present invention is used in combination with other cancer treatments for treating cancer, said other cancer treatments are selected from the group consisting of chemotherapy, immunotherapy, radiation therapy and surgery.

In another aspect, the present invention provides a method for treating cancer in a subject in need, including administering to the subject a therapeutic effective amount of the nucleic acid molecule, the small RNA molecule, the precursor miRNA, the polynucleotide, the expression carrier, the host cell or the pharmaceutical composition according to the present invention.

Unless stated otherwise, the terms used herein have the meanings generally understood by those skilled in the art.

In another aspect, provided is a method for treating a disease associated with abnormal gene expression, including administering to a subject a therapeutic effective amount of the nucleic acid molecule, the small RNA molecule, the precursor miRNA, the polynucleotide, the expression carrier, the host cell or the pharmaceutical composition according to the present invention.

In another aspect, the present invention provides a method for inhibiting the expression and/or activity of a gene, including administering to the subject a therapeutic effective amount of the nucleic acid molecule, the small RNA molecule, the precursor miRNA, the polynucleotide, the expression carrier, the host cell or the pharmaceutical composition according to the present invention.

### Terms

Generally, siRNA, miRNA and other non-coding small RNAs are indiscriminately referred to as oligonucleotides or small RNAs (sRNAs). As used herein, "small RNAs" are a large group of small, non-coding RNAs encoded within animal and plant genomes, with a length of about 18-24 nucleotides. Studies have shown that small RNAs are involved in a wide variety of regulatory pathways, including development, viral defense, hematopoietic processes, organ formation, cell proliferation and apoptosis, fat metabolism, etc.

As used herein, a small RNA (sRNA) can be a single-stranded or double-stranded RNA, including but not limited to siRNA and miRNA, which may be a natural or synthetic RNA.

As used herein, the term "nucleic acid" includes "polynucleotide", "oligonucleotide" and "nucleic acid molecule", and generally refers to DNA or RNA polymers, which may be single-stranded or double-stranded, synthetic or obtained from natural sources (e.g., isolated and/or purified); it may comprise natural, unnatural, or altered nucleotides. In some embodiments, a nucleic acid does not comprise any insertion, deletion, inversion, and/or substitution. However, as discussed herein, in some cases it may be appropriate for a nucleic acid to comprise one or more insertions, deletions, inversions, and/or substitutions.

As used herein, the term "hybridizing under stringent conditions" means that a nucleotide sequence specifically hybridizes to a target sequence (e.g., a sequence as shown in SEQ ID NO: 1) in an amount that is detectably stronger than non-specific hybridization. Stringent conditions can include, for example, low salt and/or high temperature conditions, such as those provided by about 0.02 M to 0.1 M of NaCl or equivalent substances at a temperature of about 50°C to 70°C.

As used herein, "sequence identity" refers to the sequence similarity between two polynucleotide sequences. When the positions in the two sequences aligned are occupied by the same base, for example if each position of two DNA molecules is occupied by adenine, then the molecules are identical at that position. The identity percentage between two sequences is a function of the number of matching or homologous positions shared by the two sequences divided by the number of positions compared × 100.

As used herein, the term "vector" refers to a recombinant expression vector that incorporates the nucleic acid described herein. The recombinant expression vector may be any suitable recombinant expression vector and may be used to transform or transfect any suitable host cell, including but not limited to plant expression vector, animal expression vector, viral vector such as retroviral vector or lentiviral vector. These vectors are well known to those skilled in the art and are commercially available.

As used herein, the term "host cell" refers to any type of cell that can be transfected with a recombinant expression vector according to the present invention. The host cell can be an eukaryotic cell, such as plant, animal, fungus, or alga, or it can be a prokaryotic cell, such as bacterium or protozoan.

A variety of transfection techniques are well known in the art, including but not limited to calcium phosphate co-precipitation, direct microinjection into cultured cells, electroporation, liposome-mediated gene transfer, lipid-mediated transduction, and nucleic acid delivery using high-speed microprojectiles.

As used herein, "treating" or "treatment" includes treating a disease state in a mammal, particularly in a human, and includes: (a) suppressing the disease state, i.e., preventing its progression; and/or (b) alleviating the disease state, i.e., causing the disease state to regress.

As used herein, the term "subject" refers to any human or non-human organism that may potentially benefit from treatment with the nucleic acid molecule of the present invention or the vector, cell, or composition comprising the same. Exemplary subjects include subjects with diabetes. Preferably, the term "subject" as used herein is a vertebrate, preferably a mammal, even more preferably a domestic animal or a companion animal, such as chicken, goose, duck, goat, sheep, cattle, pig, horse, dog, cat, hamster, rat, mouse, hamster, or guinea pig. Most preferably, the subject is a human.

As used herein, the term "therapeutic effective amount" is intended to include the amount of the nucleic acid molecule of the present invention or the vector, cell or composition comprising the same, which would benefit a subject when the nucleic acid molecule of the present invention or the vector, cell or composition comprising the same is administered alone or in combination.

As used herein, the term "complementary binding" or "binding complementarily" means that the two single strands form a detectable double strand by base pairing with each other. However, as long as a stable double strand can be formed, a certain percent of mismatches between the two single strands is allowed. For example, in some embodiments, the double strand has about 1%, about 2%, about 3%, about 4%, about 5%, about 6%, about 7%, about 8%, about 9%, about 10%, about 15%, about 20%, about 25%, about 30%, about 35%, about 40%, about 45%, or about 50% of mismatches.

The specific embodiments below are provided in the present specification in order to illustrate the present invention in more details, and are illustrated with reference to the attached drawings, but the solutions of the present disclosure is not limited thereto. Those skilled in the art may, on the basis of the general knowledge in the art, make appropriate alterations to the method, use and small RNA of the present invention, which should fall within the scope of the present invention as long as they can realize the functions described in the present invention.

### DESCRIPTION OF THE DRAWINGS

Figure 1A to Figure 1C: The effect of transfection of 31 small RNAs into lung cancer cells on the cell viability thereof, as detected by MTS assay.
Figure 2A to Figure 2I: Anti-cell viability effects in a variety of cancer cell lines.
Figure 3A to Figure 3H: The effect of transfection of 116 small RNAs into pancreatic cancer cells on the cell viability thereof, as detected by MTS assay.
Figure 4A and Figure 4B: The concentration-dependent activity of HQi-sRNA-2 on H460 and A549 cells.
Figure 5A: HQi-sRNA-2 inhibits the migration and invasion of H460 cells, and promotes apoptosis of H460 cells. Figure 5A shows the results of the Celigo cell scratch assay; Figure 5B shows the results of cell migration; Figure 5C shows inhibition of cell invasion; Figure 5D shows the results of pro-apoptosis.
Figure 6: HQi-sRNA-2 significantly prolongs the survival of mice with primary lung cancer.
Figure 7: HQi-sRNA-2 significantly maintains the body weight of mice with primary lung cancer.
Figure 8A and Figure 8B: HQi-sRNA-2 significantly reduces the tumor burden of mice with primary lung cancer.
Figure 9A and Figure 9B: HQi-sRNA-2 significantly increases tumor tissue apoptosis of mice with primary lung cancer.
Figure 10A and Figure 10B: MDM-sRNA-39 inhibits the migration of lung cancer H460 cells.
Figure 11A and Figure 11B: MDM-sRNA-39 inhibits the invasion of lung cancer H460 cells.
Figure 12: MDM-sRNA-39 promotes the apoptosis of lung cancer H460 cells.
Figure 13A and Figure 13B: MDM-sRNA-163 inhibits the migration of lung cancer H460 cells.
Figure 14A and Figure 14B: MDM-sRNA-163 inhibits the invasion of lung cancer H460 cells.
Figure 15: MDM-sRNA-163 promotes the apoptosis of lung cancer H460 cells.
Figure 16A and Figure 16B: MDM-sRNA-39 inhibits the activity of lung cancer H460 and A549 cells in a concentration-dependent manner.
Figure 17A and Figure 17B: MDM-sRNA-163 inhibits the activity of lung cancer H460 and A549 cells in a concentration-dependent manner.
Figure 18A and Figure 18B: MDM-sRNA-39 and MDM-sRNA-163 significantly reduce the tumor burden of mice with primary lung cancer.
Figure 19: MDM-sRNA-39 and MDM-sRNA-163 significantly prolong the survival of mice with primary lung cancer.
Figure 20A to Figure 20F: MDM-sRNA-39 inhibits the activity of breast cancer MCF7 cells, breast cancer MDA-MB-231 cells, gastric cancer AGS cells, pancreatic cancer HS766T cells, pancreatic cancer ASPC-1 cells, and prostate cancer PC-3 cells in a concentration-dependent manner.
Figure 21A to Figure 21F: MDM-sRNA-163 inhibits the activity of breast cancer MCF7 cells, breast cancer MDA-MB-231 cells, gastric cancer AGS cells, pancreatic cancer HS766T cells, pancreatic cancer ASPC-1 cells, and prostate cancer PC-3 cells in a concentration-dependent manner.
Figure 22A to Figure 22B: MDM-sRNA-39 promotes the apoptosis of pancreatic cancer PANC-1 cells and BXPC-3 cells.
Figure 23A to Figure 23D: MDM-sRNA-39 inhibits the migration of pancreatic cancer ASPC-1 cells and BXPC-3 cells.
Figure 24A to Figure 24D: MDM-sRNA-39 inhibits the invasion of pancreatic cancer ASPC-1 cells and BXPC-3 cells.
Figures 25A to Figure 25B: MDM-sRNA-163 promotes the apoptosis of pancreatic cancer PANC-1 cells and BXPC-3 cells.
Figures 26A to Figure 26D: MDM-sRNA-163 inhibits the migration of pancreatic cancer ASPC-1 cells and BXPC-3 cells.
Figures 27A to Figure 27B: MDM-sRNA-163 inhibits the invasion of pancreatic cancer BXPC-3 cells.
Figure 28: MDM-sRNA-39 promotes the apoptosis of breast cancer MCF7 cells.
Figure 29: MDM-sRNA-39 promotes the apoptosis of prostate cancer PC-3 cells.
Figure 30: MDM-sRNA-163 promotes the apoptosis of breast cancer MCF7 cells.
Figure 31: MDM-sRNA-163 promotes the apoptosis of prostate cancer PC-3 cells.
Figure 32: The functions of genes down-regulated at transcriptional level by HQi-sRNA-2 are mainly clustered in oncology, immunology and other related biological pathways.
Figure 33: The functions of genes down-regulated at transcriptional level by MDM-sRNA-39 are mainly clustered in oncology, immunology and other related biological pathways.
Figure 34: The functions of genes down-regulated at transcriptional level by MDM-sRNA-163 are mainly clustered in oncology, immunology, and other related biological pathways.
Figure 35A to Figure 35D: HQi-sRNA-2 can significantly down-regulate the mRNA and protein levels of cyclooxygenase-2 (COX2) in H460 cells and lung tissues of mice with lung cancer.
Figure 36: Luciferase reporter assay demonstrates that HQi-sRNA-2 can down-regulate cyclooxygenase-2 (COX2) expression, confirming that COX2 is a target of HQi-sRNA-2.

The examples are incorporated below for further description of the present invention, but these examples do not limit the scope of the present invention. The experimental methods with unspecified conditions in the examples of the present invention generally follow conventional conditions, or according to the conditions recommended by the raw material or commodity manufacturer. The reagents without specified sources are conventional reagents purchased from the market.

As for the small RNAs used in the following examples, the ones used in cellular experiments are double-stranded RNA sequences synthesized by complementation of the provided sequence, and the ones used in animal experiments are single-stranded small RNA sequences.

### DETAILED DESCRIPTION OF THE INVENTION

### Example 1. Verification of the proliferation-inhibiting function of small RNAs derived from traditional Chinese medicine on H460, H23 and A549 cells

1) H460, H23 and A549 cells with a cell density of more than 90% in 10 cm2 dishes were plated in 96-well plates according to the cell growth rate, and transfected after 12 h, and the groups (3 wells per group) were divided as follows:
   blank control: 20 ul of opti-MEM (serum-free medium)
   negative control group (NC group) and experimental group:
      A. 10 ul of opti-MEM and 0.2 ul of iMax (RNAimax transfection reagent) were mixed well for 5 min
      B. 10 ul of opti-MEM and 0.5 ul of small RNA (final concentration 100 nM/L) were mixed well
      C. solution A and solution B were mixed well and let stand for 15 min, and then was added at a 20 ul/well system
   positive group: the concentration of paclitaxel was adjusted to 100 nM/L
   Note: 100 ul of nuclease-free water was added to 2 nmol of small RNA dry powder for dissolution at a storage concentration of 20 uM/L
2) Detection of cell viability: MTS detection was performed after 48 h.

Formulation of MTS detection solution reagent (G1112, promega): opti-MEM: MTS A solution: MTS B solution = 100:20:1. After removing the supernatant, 100 ul of MTS detection solution was added to each well and incubated for 0.5-2 h. Absorbance values at wavelengths of 490 nm and 630 nm were measured in a microplate reader.

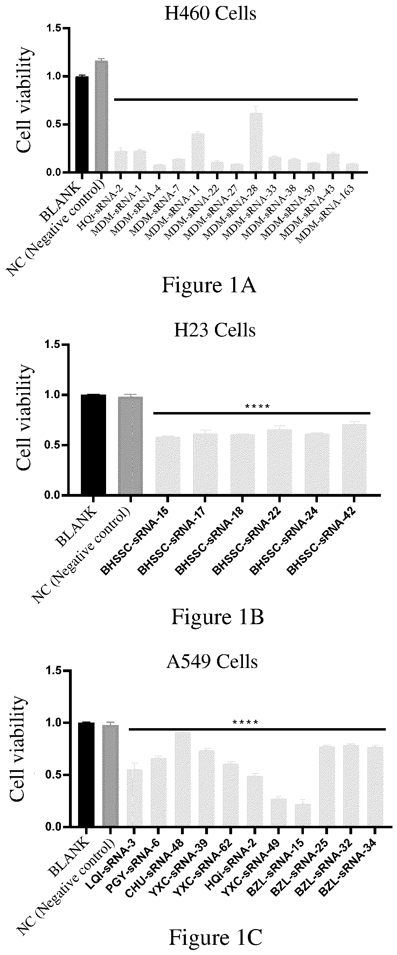

The results were as shown in Figure 1A to Figure 1C.

### Example 2. Verification of the proliferation-inhibiting function of small RNAs derived from traditional Chinese medicine in various cell lines

1) PC3, MCF7, MDA-MB-231, MKN45, HCT116, AGS, A549, HS766T and ASPC1 cells with a cell density of more than 90% in 10 cm2 dishes were plated in 96-well plates according to the cell growth rate, and transfected after 12 h, and the groups (3 wells per group) were divided as follows:
   blank control group: 20 ul of opti-MEM
   negative control group (NC group) and experimental group:
      A. 10 ul of opti-MEM and 0.2 ul of iMax were mixed well for 5 min
      B. 10 ul of opti-MEM and 0.5 ul of small RNA (final concentration 100 nM/L) were mixed well
      C. solution A and solution B were mixed well and let stand for 15 min, and then was added at a 20 ul/well system
   positive group: the concentration of paclitaxel was adjusted to 100 nM/L
   Note: 100 ul of nuclease-free water was added to 2 nmol of small RNA dry powder for dissolution at a storage concentration of 20 uM/L
2) Detection of cell viability: MTS detection was performed after 48 h.

Formulation of MTS detection solution: opti-MEM: MTS A solution: MTS B solution = 100:20:1. After removing the supernatant, 100 ul of MTS detection solution was added to each well and incubated for 0.5-2 h. Absorbance values at wavelengths of 490 nm and 630 nm were measured in a microplate reader.

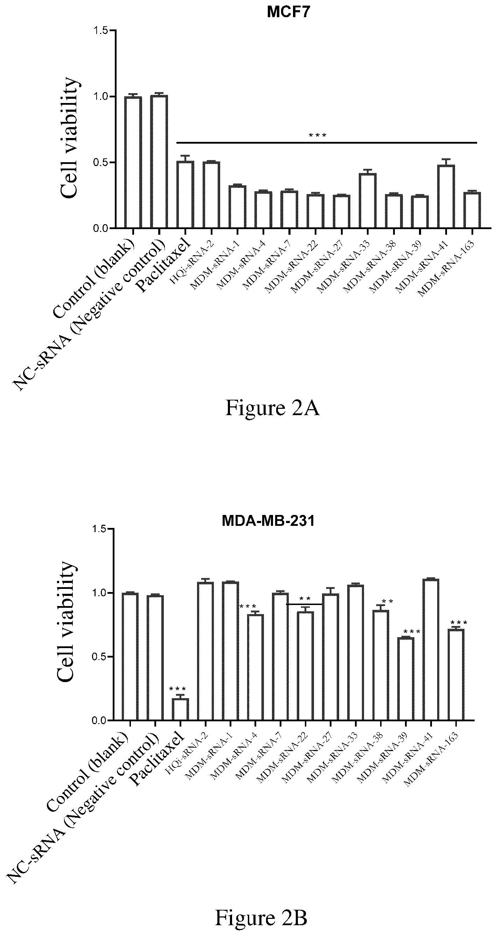

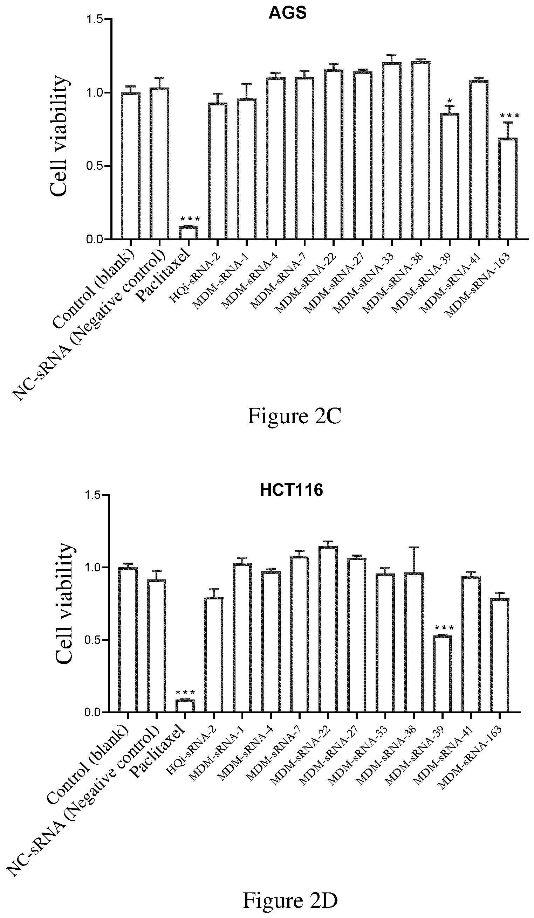

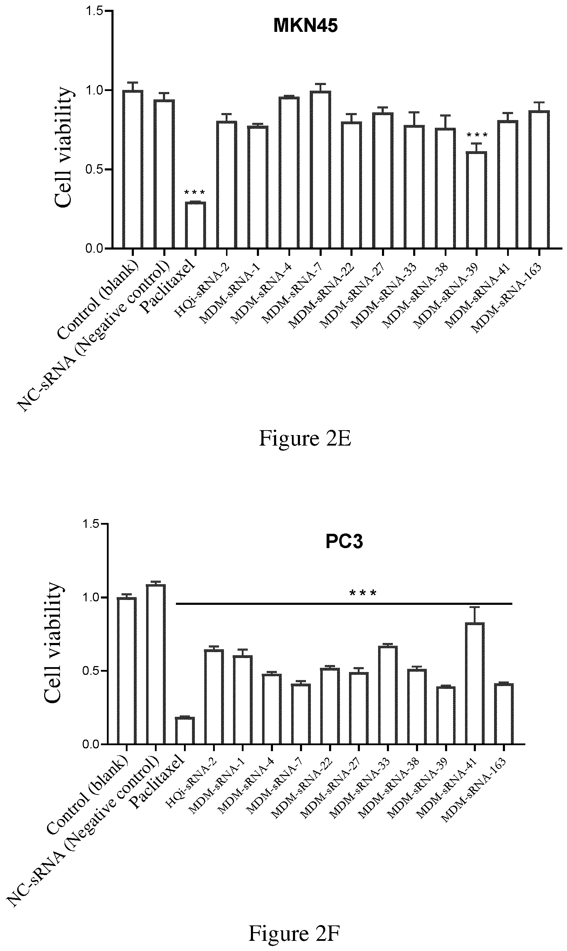

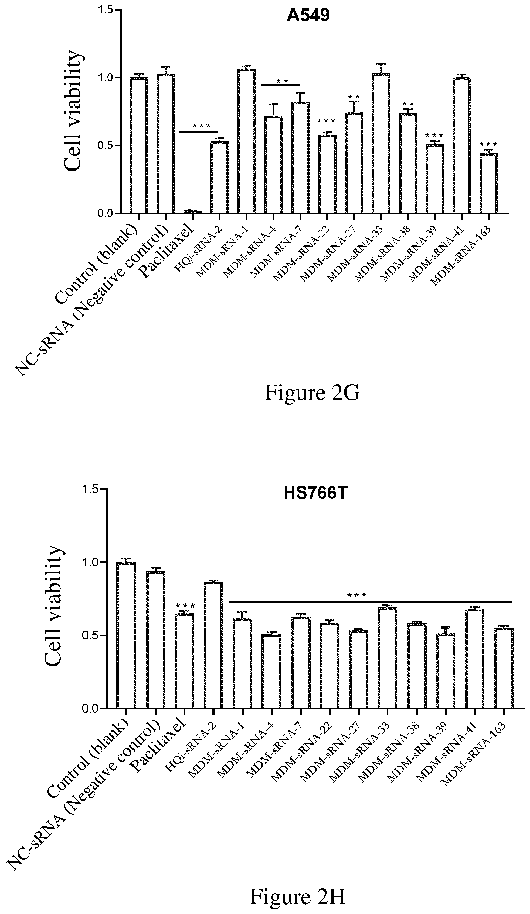

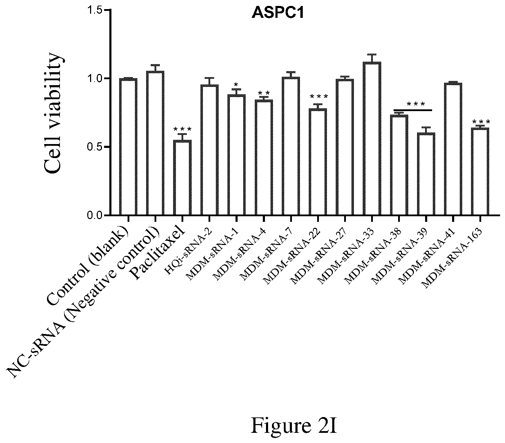

The results were as shown in Figure 2A to Figure 2I.

### Example 3. Verification of the proliferation-inhibiting function of small RNAs derived from traditional Chinese medicine in pancreatic cancer cell lines

1) PANC-1 and HS 766T cells with a cell density of more than 90% in 10cm² dishes were plated in 96-well plates according to the cell growth rate, and transfected after 12 h, and the groups (3 wells per group) were divided as follows:
   blank control group: 20 ul of opti-MEM
   negative control group (NC group) and experimental group:
      A. 10 ul of opti-MEM and 0.2 ul of iMax were mixed well for 5 min
      B. 10 ul of opti-MEM and 0.5 ul of small RNA (final concentration 100 nM/L) were mixed well
      C. solution A and solution B were mixed well and let stand for 15 min, and then was added at a 20 ul/well system
   positive group: the concentration of paclitaxel was adjusted to 100 nM/L
   Note: 100 ul of nuclease-free water was added to 2 nmol of small RNA dry powder for dissolution at a storage concentration of 20 uM/L
2) Detection of cell viability: MTS detection was performed after 48 h.

Formulation of MTS detection solution: opti-MEM: MTS A solution: MTS B solution = 100:20:1. After removing the supernatant, 100 ul of MTS detection solution was added to each well and incubated for 0.5-2 h. Absorbance values at wavelengths of 490 nm and 630 nm were measured in a microplate reader.

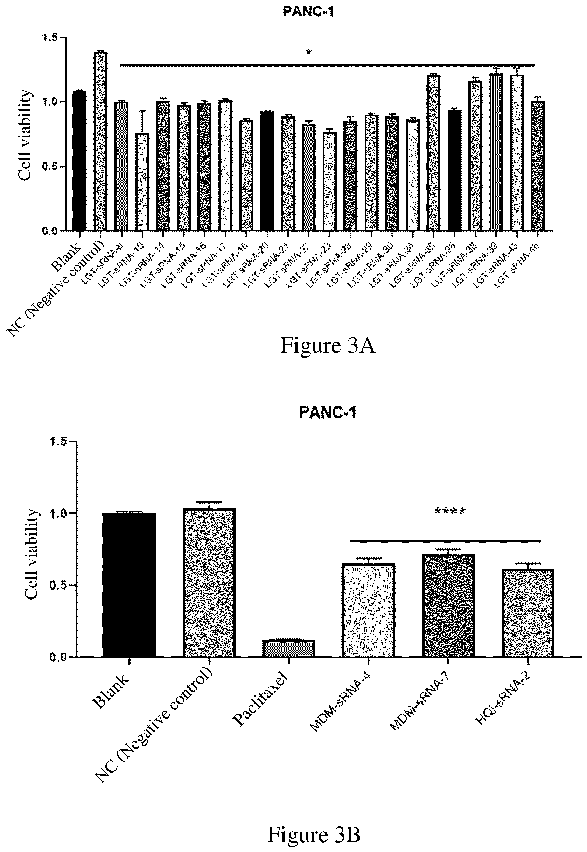

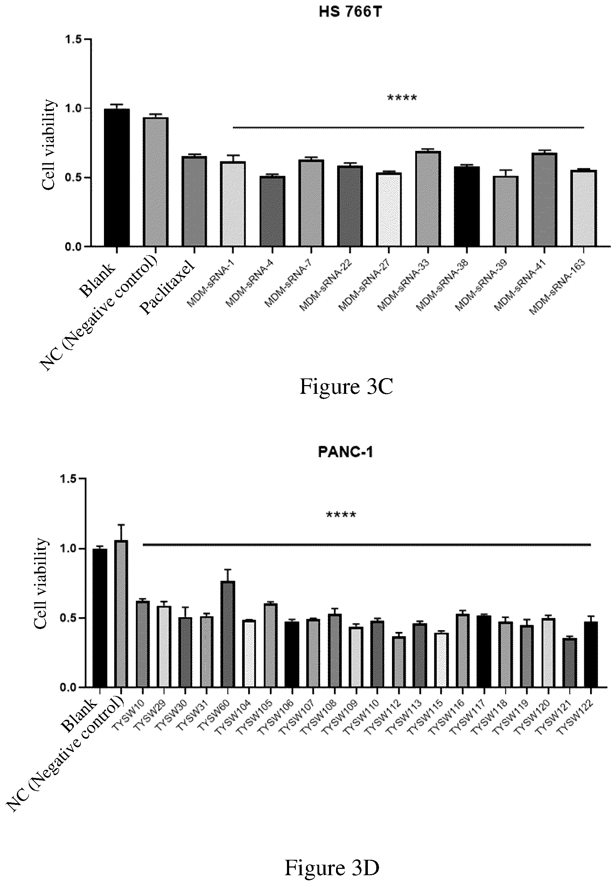

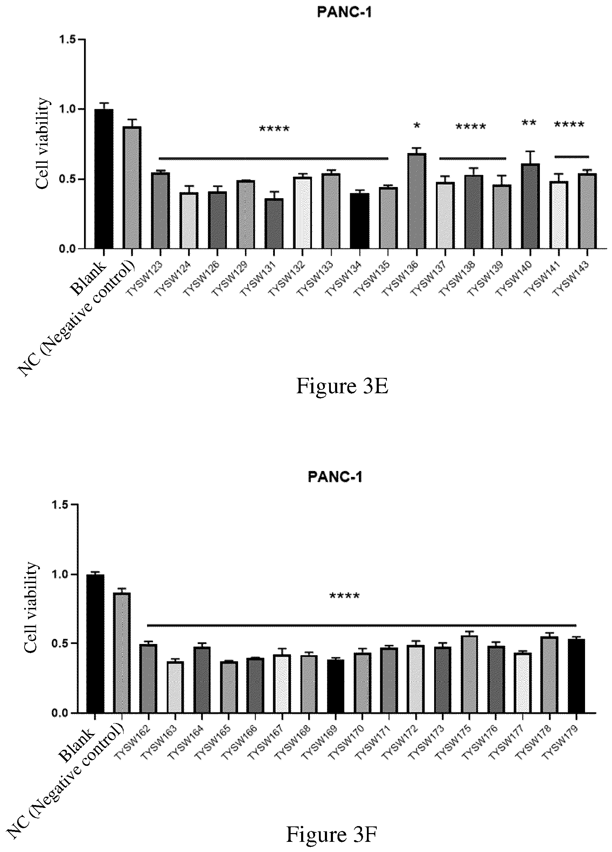

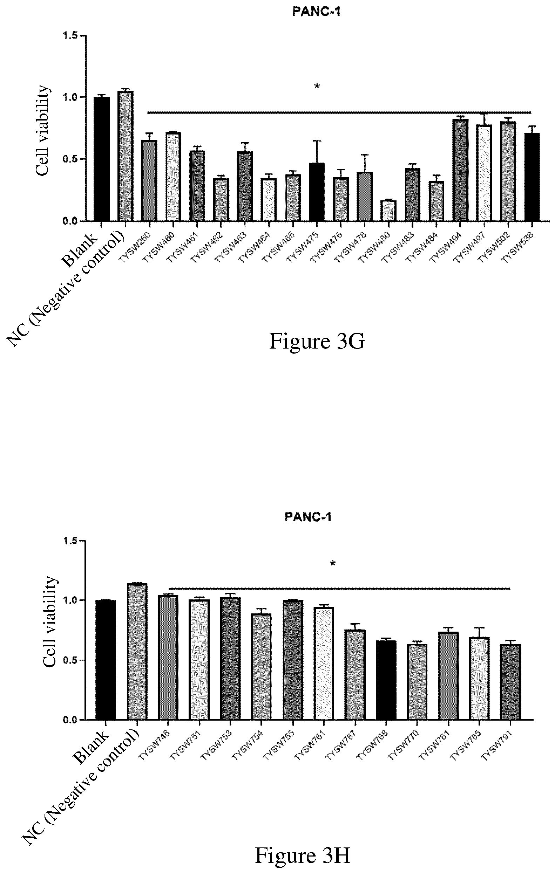

The results were as shown in Figure 3A to Figure 3H.

### Example 4. Verification of concentration-dependent proliferation-inhibiting function of sRNA-2 derived from Scutellaria baicalensis on H460 and A549 cells

1) H460 and A549 cells with a cell density of more than 90% in 10cm² dishes were plated in 96-well plates according to the cell growth rate, and transfected after 12 h, and the groups (3 wells per group) were divided as follows:
   BLANK group: 20uL of opti-MEM
   negative control group (NC group) and experimental group:
      A. 10 uL of opti-MEM and 0.2 uL of iMax were mixed well for 5 min
      B. 10 uL of opti-MEM and small RNA were mixed well (the concentration gradient of H460 cell experimental group was set as: 0.01 noml/L, 0.03 noml/L, 0.1 noml/L, 0.39 noml/L, 0.78 noml/L, 1.56 noml/L, 3.125 noml/L, 6.25 noml/L, 12.5 noml/L, 25 noml/L, 50 noml/L, 100 noml/L; the concentration gradient of A549 cell experimental group was set as: 0.01 noml/L, 0.03 noml/L, 0.1 noml/L, 0.3 noml/L, 1 noml/L, 3 noml/L, 10 noml/L, 30 noml/L, 100 noml/L, 300 noml/L; the final concentration of sRNA in NC group was 100 noml/L)
      C. solution A and solution B were mixed well and let stand for 15 min, and then was added at a 20 ul/well system
   Note: 100 uL of nuclease-free water was added to 2 nmol of small RNA dry powder for dissolution at a storage concentration of 20 umol/L
2) Detection of cell viability: MTS detection was performed after 48 h.

Formulation of MTS detection solution was as follows: opti-MEM: MTS A solution: MTS B solution = 100:20:1. After removing the supernatant, 100 uL of MTS detection solution was added to each well and incubated for 0.5-2 h. Absorbance values at wavelengths of 490 nm and 630 nm were measured in a microplate reader. It was found that the proliferation-inhibiting function of HQi-sRNA-2 derived from *Scutellaria baicalensis* on H460 and A549 cells was concentration-dependent.

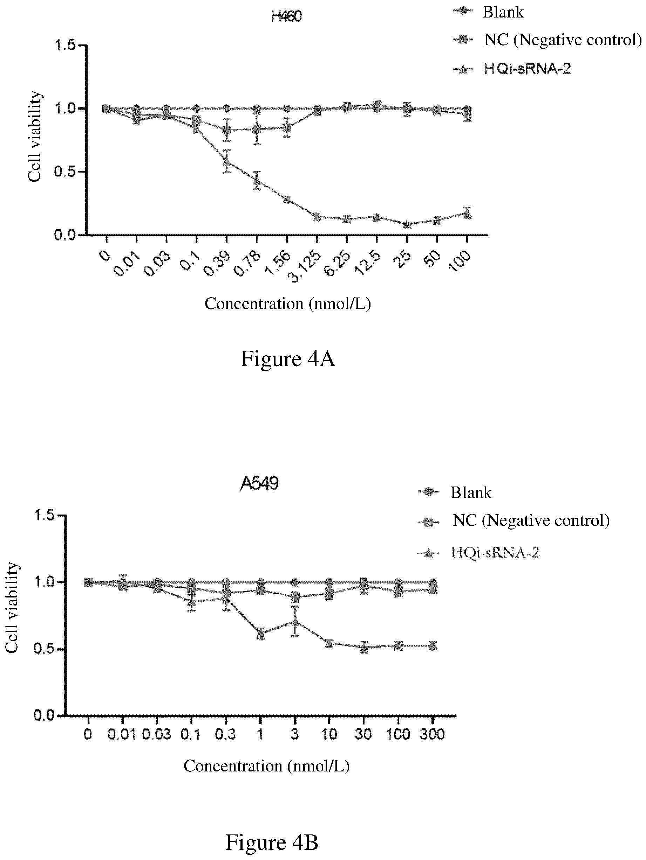

The results were as shown in Figure 4A to Figure 4B.

### Example 5. Verification of migration- and invasion-inhibiting function as well as pro-apoptotic function of sRNA-2 derived from Scutellaria baicalensis on H460 cells

### 1) Migration-inhibiting function

Method 1: H460 cells with a cell density of more than 90% in 10 cm² dishes were plated in 12-well plates using serum-free medium according to the cell growth rate. After 12 h, 200 uL pipette tips were used to draw straight lines in the wells. The floating cells were washed away and transfection was performed, and the groups (three wells per group, two lines per well) were divided as follows:
blank control group: 200 uL of opti-MEM per well
negative control group (NC group) and experimental group:
   A. 100 uL of opti-MEM and 2 uL of iMax were mixed well for 5 min
   B. 100 uL of opti-MEM and 5 uL of small RNA (final concentration 100 nmol/L) were mixed well
   C. solution A and solution B were mixed well and let stand for 15 min, and then was added to each well at a 200 uL system
Note: 100 uL of nuclease-free water was added to 2 nmol of small RNA dry powder for dissolution at a storage concentration of 20 umol/L

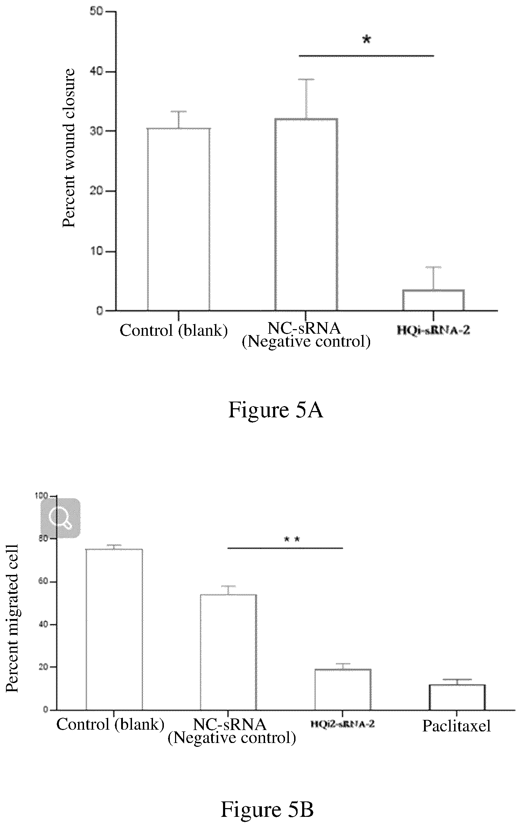

After 48 h, the supernatant was discarded and images were taken under a microscope. The results were as shown in Figure 5A.

Method 2: H460 cells with a cell density of more than 90% in 10 cm² dishes were digested and then resuspended with 1 mL of serum-free medium and counted. 100,000 cells (100 uL) were plated in the upper chamber, and complete medium with 10% FBS was added to the lower chamber of each Transwell (migration chamber). Transfection was performed after 12 h, and the groups (3 wells per group) were divided as follows:
blank control group: 20 uL of opti-MEM
negative control group (NC group) and experimental group:
   A. 10 uL of opti-MEM and 0.2 uL of iMax were mixed well for 5 min
   B. 10 uL of opti-MEM and 0.5 uL of small RNA (final concentration 100 nmol/L) were mixed well
   C. solution A and solution B were mixed well and let stand for 15 min, and then was added to each well at a 20 uL system
Note: 100 uL of nuclease-free water was added to 2 nmol of small RNA dry powder for dissolution at a storage concentration of 20 umol/L

After 48 h, the medium in upper and lower chambers was discarded. The cells were fixed with paraformaldehyde at room temperature for 15 min, stained with 0.1% crystal violet at room temperature for 15 min, and rinsed with PBS. The cells in the upper chamber were gently wiped off with cotton swabs, and the chamber was dried. A Leica inverted microscope DMi8 was turned on, the 5× objective lens was selected, the parameters were adjusted and the white balance was performed following the manual. Each chamber was photographed, followed by counting and statistic processing. The results were as shown in Figure 5B.

### 2) Invasion-inhibiting function

Matrigel (basement membrane matrix) was thawed on crushed ice one night before and diluted with serum-free medium to a final concentration of 200-300 ug/mL. 100 uL of the diluted Matrigel was added to each upper chamber, let stand at 37°C for 30 min, and the supernatant was aspirated. H460 cells with a cell density of more than 90% in 10 cm² dishes were digested and resuspended with 1 mL of serum-free medium and counted. 100,000 cells (100 uL) were plated in the upper chamber, and complete medium with 10% FBS was added to the lower chamber of each chamber. Transfection was performed after 12 h, and the groups (3 wells per group) were divided as follows:
blank control group: 20 uL of opti-MEM
negative control group (NC group) and experimental group:
   A. 10 uL of opti-MEM and 0.2 uL of iMax were mixed well for 5 min
   B. 10 uL of opti-MEM and 0.5 uL of small RNA (final concentration 100 nmol/L) were mixed well
   C. solution A and solution B were mixed well and let stand for 15 min, and then was added to each well at a 20 uL system
Note: 100 uL of nuclease-free water was added to 2 nmol of small RNA dry powder for dissolution at a storage concentration of 20 umol/L

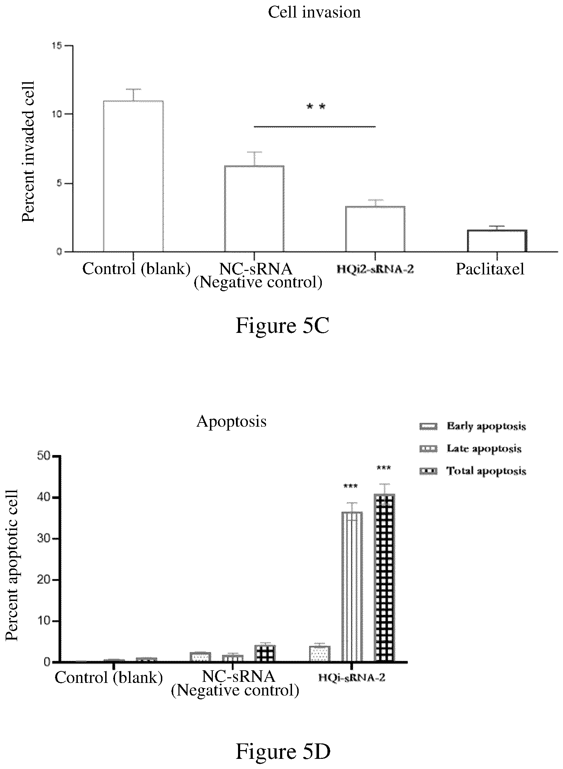

After 48 h, the medium in upper and lower chambers was discarded. The cells were fixed with paraformaldehyde at room temperature for 15 min, stained with 0.1% crystal violet at room temperature for 15 min, and rinsed with PBS. The cells in the upper chamber were gently wiped off with cotton swabs, and the chamber was dried. A Leica inverted microscope DMi8 was turned on, the 5× objective lens was selected, the parameters were adjusted and the white balance was performed following the manual. Each chamber was photographed, followed by counting and statistic processing. The results were as shown in Figure 5C.

### 3) Pro-apoptotic function

H460 cells with a cell density of more than 90% in 10 cm² dishes were plated in 12-well plates according to the cell growth rate, and transfected after 12 h, and the groups were divided as follows:
blank control group (4 wells): 200 uL of opti-MEM per well
negative control group (NC group) and experimental group (3 wells each): the system in each well was as follows
   A. 100 uL of opti-MEM and 2 uL of iMax were mixed well for 5 min
   B. 100 uL of opti-MEM and 5 uL of small RNA (final concentration 100 nmol/L) were mixed well
   C. solution A and solution B were mixed well and let stand for 15 min, and then was added to each well at a 200 uL system
Note: 100 uL of nuclease-free water was added to 2 nmol of small RNA dry powder for dissolution at a storage concentration of 20 umol/L

The supernatant and cells were collected after 48 h and washed twice with pre-chilled PBS. The 4 tubes of cells in the blank control group were added respectively with: (1) 200 uL of 1× binding buffer; (2) 200 uL of 1× binding buffer and 5 uL of Annexin V stain; (3) 200 uL of 1× binding buffer and 5 uL of PI stain; (4) 200 uL of 1× binding buffer, 5 uL of Annexin V stain and 5 uL of PI stain. 200 uL of 1× binding buffer, 5 uL of Annexin V stain and 5 uL of PI stain were added to each tube of both the NC group and the experimental group. The mixture was mixed well and filtered through 300 mesh filters. The live cell population and the position of quadrant gate were selected according to the blank control group, and then the apoptosis of the NC group and the experimental group was detected. The results were as shown in Figure 5D.

### Example 6. HQi-sRNA-2 significantly prolonged survival of mice with primary lung cancer

The mouse model of primary lung cancer was Kras^{LSL-G12D}p53^{fl/fl} (KP).

Mice were weighed and assigned to the negative control group (NC group) and HQi-sRNA-2 group randomly. The NC group was the negative control, in which a sphingosine(d18:1)-NC-sRNA herbal medicine delivery system was constructed from NC-sRNA. The HQi-sRNA-2 group was the experimental group, in which a sphingosine(d18:1)-HQi-sRNA-2 herbal medicine delivery system was constructed from HQi-sRNA-2 and sphingosine(d18:1).

The herbal medicine delivery system was constructed by heating and mixing 10 nmol of sRNA, 100 ug of sphingosine(d18:1) and 300 ul of sterile enzyme-free water in a water bath at 90°C for 15 minutes.

Each mouse with a uniform body weight was dosed by gavage. Each mouse was given 10 nmol of HQi-sRNA-2 mimic daily by gavage until death of the mouse.

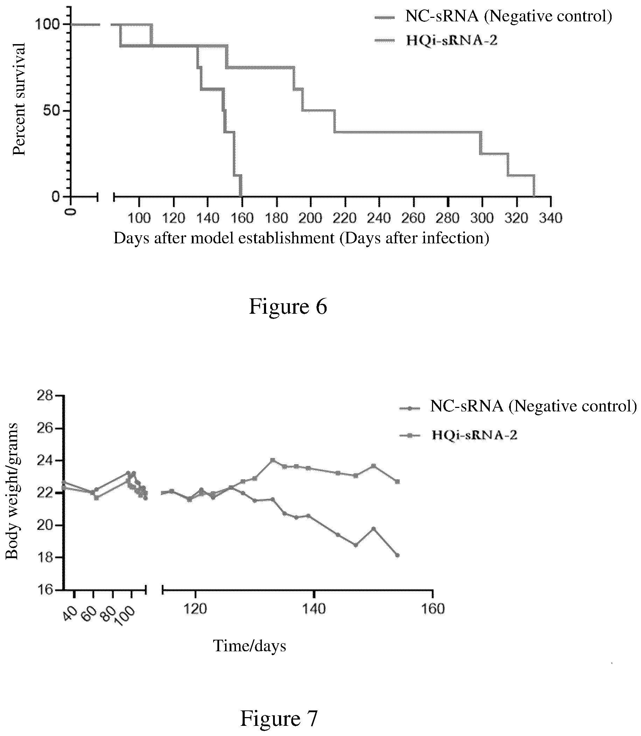

The survival time of each mouse was recorded, the survival curve was plotted, and statistical analysis was performed. It was found that HQi-sRNA-2 significantly prolonged the survival of mice with primary lung cancer. The results were as shown in Figure 6.

### Example 7. HQi-sRNA-2 significantly maintained body weight of mice with primary lung cancer

The mouse model of primary lung cancer was Kras^{LSL-G12D}p53^{fl/fl} (KP).

Mice were weighed and assigned to the negative control group (NC group) and HQi-sRNA-2 group randomly. The NC group was the negative control, in which a sphingosine(d18:1)-NC-sRNA herbal medicine delivery system was constructed from NC-sRNA. The HQi-sRNA-2 group was the experimental group, in which a sphingosine(d18:1)-HQi-sRNA-2 herbal medicine delivery system was constructed from HQi-sRNA-2.

The herbal medicine delivery system was constructed by heating and mixing 10 nmol of sRNA, 100 ug of sphingosine(d18:1) and 300 ul of sterile enzyme-free water in a water bath at 90°C for 15 minutes.

Each mouse with a uniform body weight was dosed by gavage. Each mouse was given 10 nmol of HQi-sRNA-2 mimic daily by gavage for one month.

The body weight of each mouse was recorded, the curve of body weight over time was plotted, and statistical analysis was performed. It was found that HQi-sRNA-2 significantly maintained the body weight of mice with primary lung cancer. The results were as shown in Figure 7.

### Example 8. HQi-sRNA-2 significantly reduced tumor burden in mice with primary lung cancer

The mouse model of primary lung cancer was Kras^{LSL-G12D}p53^{fl/fl} (KP).

Mice were weighed and assigned to the negative control group (NC group) and HQi-sRNA-2 group randomly. The NC group was the negative control, in which a sphingosine(d18:1)-NC-sRNA herbal medicine delivery system was constructed from NC-sRNA. The HQi-sRNA-2 group was the experimental group, in which a sphingosine(d18:1)-HQi-sRNA-2 herbal medicine delivery system was constructed from HQi-sRNA-2.

The herbal medicine delivery system was constructed by heating and mixing 10 nmol of sRNA, 100 ug of sphingosine(d18:1) and 300 ul of sterile enzyme-free water in a water bath at 90°C for 15 minutes.

Each mouse with a uniform body weight was dosed by gavage. Each mouse was given 10 nmol of HQi-sRNA-2 mimic daily by gavage for one month.

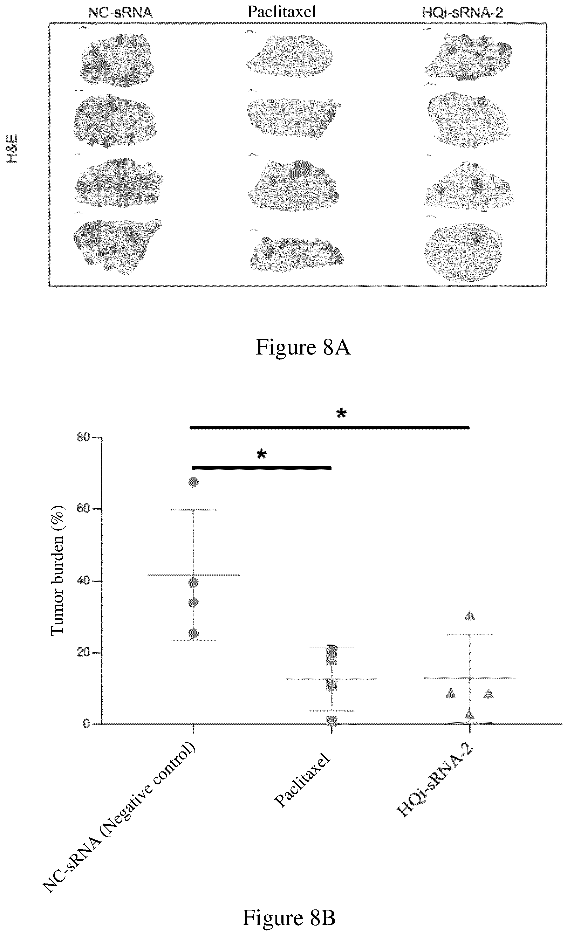

The lungs of mice were collected to prepare pathological sections. The left lung was collected and sliced at the largest surface for every mouse, and stained with HE. The area of tumor in the whole pathological section was counted, the tumor burden was calculated, and statistical analysis was performed. It was found that HQi-sRNA-2 significantly reduced the tumor burden in mice with primary lung cancer. The results were as shown in Figure 8A and Figure 8B.

### Example 9. HQi-sRNA-2 significantly increased tumor tissue apoptosis in mice with primary lung cancer

The mouse model of primary lung cancer was Kras^{LSL-G12D}p53^{fl/fl} (KP).

Mice were weighed and assigned to the negative control group (NC group) and HQi-sRNA-2 group randomly. The NC group was the negative control, in which a sphingosine(d18:1)-NC-sRNA herbal medicine delivery system was constructed from NC-sRNA. The HQi-sRNA-2 group was the experimental group, in which a sphingosine(d18:1)-HQi-sRNA-2 herbal medicine delivery system was constructed from HQi-sRNA-2.

The herbal medicine delivery system was constructed by heating and mixing 10 nmol of sRNA, 100 ug of sphingosine(d18:1) and 300 ul of sterile enzyme-free water in a water bath at 90°C for 15 minutes.

Each mouse with a uniform body weight was dosed by gavage. Each mouse was given 10 nmol of HQi-sRNA-2 mimic daily by gavage for one month.

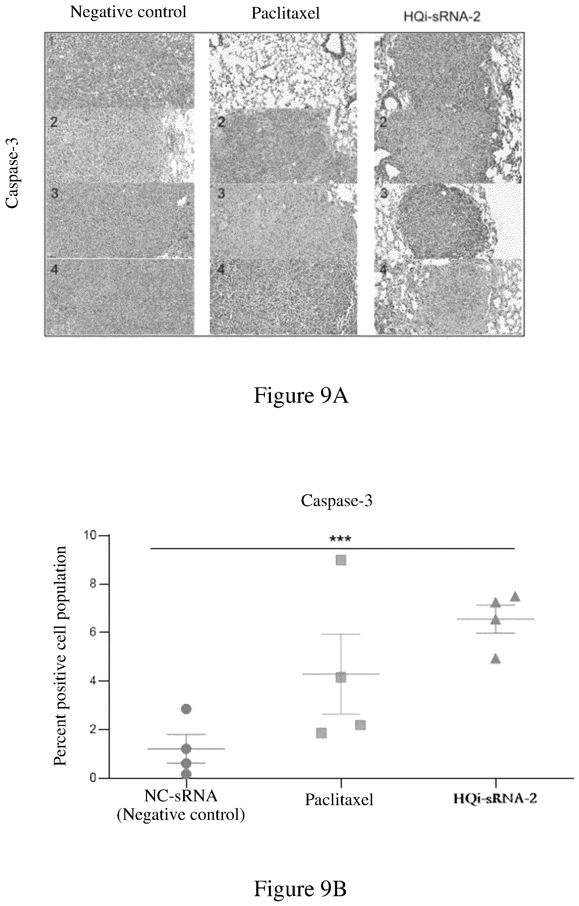

The lungs of mice were collected to prepare pathological sections. The left lung was collected and sliced at the largest surface for every mouse, and subjected to immunohistochemistry of caspase-3. The area of positive cell population in the whole pathological section was counted, the tumor burden was calculated, and statistical analysis was performed. It was found that HQi-sRNA-2 significantly increased tumor tissue apoptosis of mice with primary lung cancer. The results were as shown in Figure 9A and Figure 9B.

### Example 10. Verification of migration-inhibiting function of sRNA-39 derived from Maytenus hookeri on H460 cells

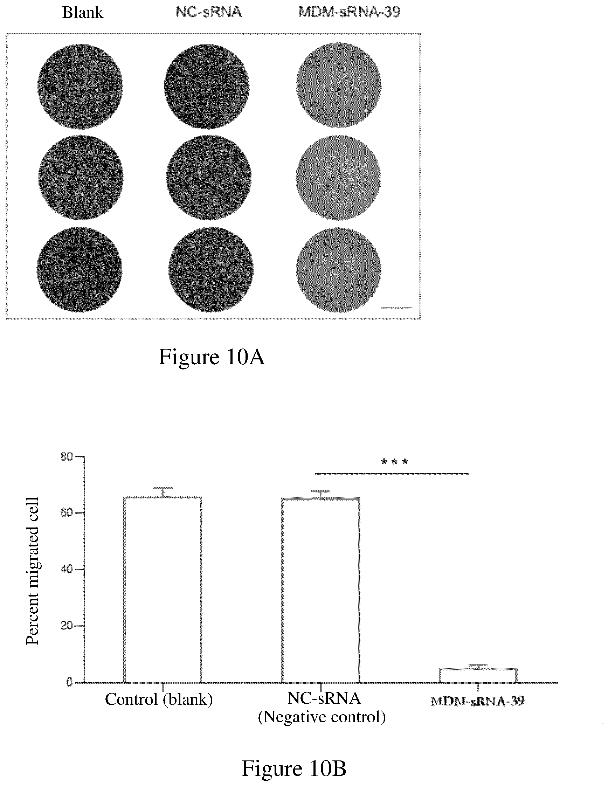

1) H460 cells with a cell density of more than 90% in 10 cm² dishes were digested and then resuspended with 1 mL of serum-free medium and counted. 100,000 cells (100 uL) were plated in the upper chamber, and complete medium with 10% FBS was added to the lower chamber of each Transwell chamber. Transfection was performed after 12 h, and the groups (3 wells per group) were divided as follows:
   blank control group: 20 uL of opti-MEM
   negative control group (NC group) and experimental group:
      A. 10 uL of opti-MEM and 0.2 uL of iMax were mixed well for 5 min
      B. 10 uL of opti-MEM and 0.5 uL of small RNA (final concentration 100 nmol/L) were mixed well
      C. solution A and solution B were mixed well and let stand for 15 min, and then was added to each well at a 20 uL system
   Note: 100 uL of nuclease-free water was added to 2 nmol of small RNA dry powder for dissolution at a storage concentration of 20 umol/L
2) Fixation, staining, photography and statistics:
   After 48 h, the medium in upper and lower chambers was discarded. The cells were fixed with paraformaldehyde at room temperature for 15 min, stained with 0.1% crystal violet at room temperature for 15 min, and rinsed with PBS. The cells in the upper chamber were gently wiped off with cotton swabs, and the chamber was dried. A Leica inverted microscope DMi8 was turned on, the 5× objective lens was selected, the parameters were adjusted and the white balance was performed following the manual. Each chamber was photographed, followed by counting and statistic processing. The results were as shown in Figure 10A and Figure 10B.

### Example 11. Verification of invasion-inhibiting function of sRNA-39 derived from Maytenus hookeri on H460 cells

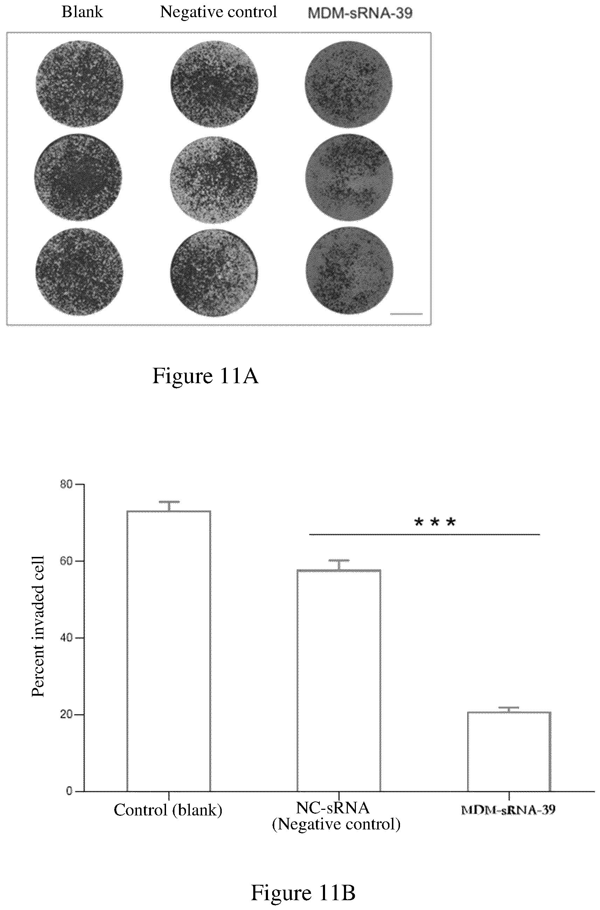

1) Matrigel basement membrane matrix was thawed on crushed ice one night before and diluted with serum-free medium to a final concentration of 200-300 ug/mL. 100 uL of the diluted Matrigel was added to each upper chamber, let stand at 37°C for 30 min, and the supernatant was aspirated. H460 cells with a cell density of more than 90% in 10 cm² dishes were digested and resuspended with 1 mL of serum-free medium and counted. 100,000 cells (100 uL) were plated in the upper chamber, and complete medium with 10% FBS was added to the lower chamber of each chamber. Transfection was performed after 12 h, and the groups (3 wells per group) were divided as follows:
   blank control group: 20 uL of opti-MEM
   negative control group (NC group) and experimental group:
      A. 10 uL of opti-MEM and 0.2 uL of iMax were mixed well for 5 min
      B. 10 uL of opti-MEM and 0.5 uL of small RNA (final concentration 100 nmol/L) were mixed well
      C. solution A and solution B were mixed well and let stand for 15 min, and then was added to each well at a 20 uL system
   Note: 100 uL of nuclease-free water was added to 2 nmol of small RNA dry powder for dissolution at a storage concentration of 20 umol/L
2) Fixation, staining, photography and statistics:
   After 48 h, the medium in upper and lower chambers was discarded. The cells were fixed with paraformaldehyde at room temperature for 15 min, stained with 0.1% crystal violet at room temperature for 15 min, and rinsed with PBS. The cells in the upper chamber were gently wiped off with cotton swabs, and the chamber was dried. A Leica inverted microscope DMi8 was turned on, the 5× objective lens was selected, the parameters were adjusted and the white balance was performed following the manual. Each chamber was photographed, followed by counting and statistic processing. The results were as shown in Figure 11A and Figure 11B.

### Example 12. Verification of pro-apoptotic function of sRNA-39 derived from Maytenus hookeri on H460 cells

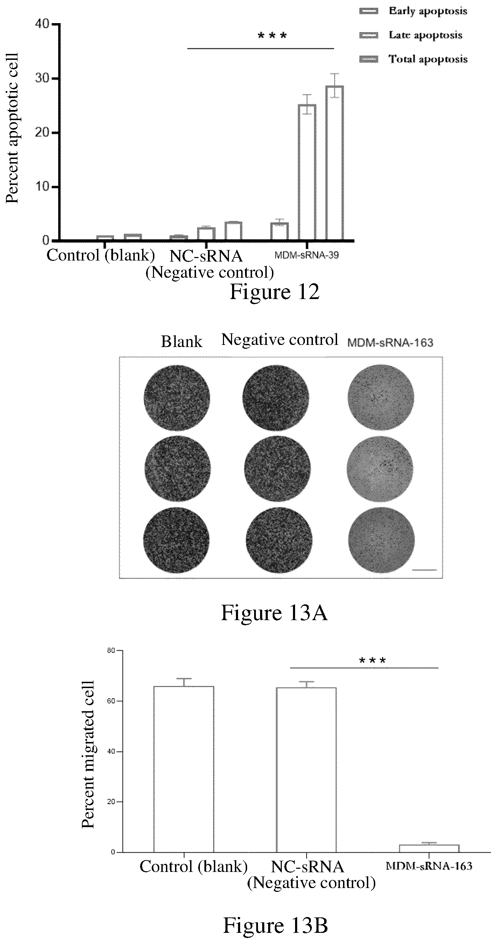

1) H460 cells with a cell density of more than 90% in 10 cm² dishes were plated in 12-well plates according to the cell growth rate, and transfected after 12 h, and the groups were divided as follows:
   blank control group (4 wells): 200 uL of opti-MEM per well
   negative control group (NC group) and experimental group (3 wells each): the system of each well was as follows
      A. 100 uL of opti-MEM and 2 uL of iMax were mixed well for 5 min
      B. 100 uL of opti-MEM and 5 uL of small RNA (final concentration 100 nmol/L) were mixed well
      C. solution A and solution B were mixed well and let stand for 15 min, and then was added to each well at a 200 uL system
   Note: 100 uL of nuclease-free water was added to 2 nmol of small RNA dry powder for dissolution at a storage concentration of 20 umol/L
2) Detection of apoptosis
   The supernatant and cells were collected after 48 h and washed twice with pre-chilled PBS. The 4 tubes of cells in the blank control group were added respectively with: (1) 200 uL of 1× binding buffer; (2) 200 uL of 1× binding buffer and 5 uL of Annexin V stain; (3) 200 uL of 1 × binding buffer and 5 uL of PI stain; (4) 200 uL of 1× binding buffer, 5 uL of Annexin V stain and 5 uL of PI stain. 200 uL of 1× binding buffer, 5 uL of Annexin V stain and 5 uL of PI stain were added to each tube of both the NC group and the experimental group. The mixture was mixed well and filtered through 300 mesh filters. The live cell population and the position of quadrant gate were selected according to the blank control group, and then the apoptosis of the NC group and the experimental group was detected. The results were as shown in Figure 12.

### Example 13. Verification of migration-inhibiting function of sRNA-163 derived from Maytenus hookeri on H460 cells

1) H460 cells with a cell density of more than 90% in 10 cm² dishes were digested and then resuspended with 1 mL of serum-free medium and counted. 100,000 cells (100 uL) were plated in the upper chamber, and complete medium with 10% FBS was added to the lower chamber of each Transwell chamber. Transfection was performed after 12 h, and the groups (3 wells per group) were divided as follows:
   blank control group: 20 uL of opti-MEM
   negative control group (NC group) and experimental group:
      A. 10 uL of opti-MEM and 0.2 uL of iMax were mixed well for 5 min
      B. 10 uL of opti-MEM and 0.5 uL of small RNA (final concentration 100 nmol/L) were mixed well
      C. solution A and solution B were mixed well and let stand for 15 min, and then a 20 uL system was added to each well
   Note: 100 uL of nuclease-free water was added to 2 nmol of small RNA dry powder for dissolution at a storage concentration of 20 umol/L
1) Fixation, staining, photography and statistics:
   After 48 h, the medium in upper and lower chambers was discarded. The cells were fixed with paraformaldehyde at room temperature for 15 min, stained with 0.1% crystal violet at room temperature for 15 min, and rinsed with PBS. The cells in the upper chamber were gently wiped off with cotton swabs, and the chamber was dried. A Leica inverted microscope DMi8 was turned on, the 5× objective lens was selected, the parameters were adjusted and the white balance was performed following the manual. Each chamber was photographed, followed by counting and statistic processing. The results were as shown in Figure 13A and Figure 13B.

### Example 14. Verification of invasion-inhibiting function of sRNA-163 derived from Maytenus hookeri on H460 cells

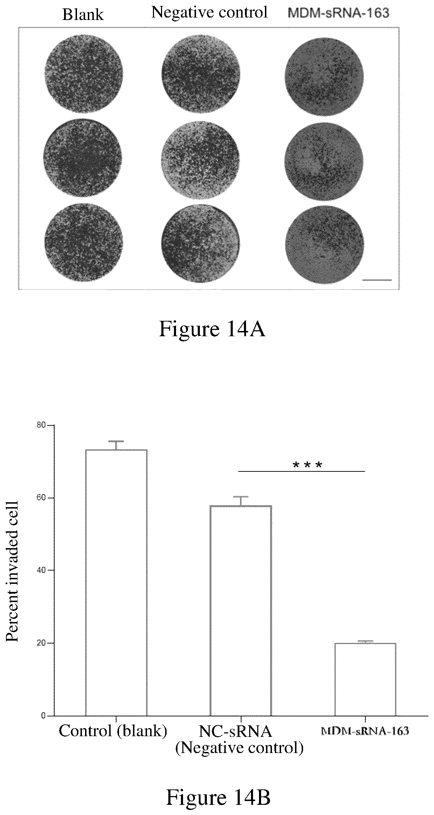

1) Matrigel basement membrane matrix was thawed on crushed ice one night before and diluted with serum-free medium to a final concentration of 200-300 ug/mL. 100 uL of the diluted Matrigel was added to each upper chamber, let stand at 37°C for 30 min, and the supernatant was aspirated. H460 cells with a cell density of more than 90% in 10 cm² dishes were digested and resuspended with 1 mL of serum-free medium and counted. 100,000 cells (100 uL) were plated in the upper chamber, and complete medium with 10% FBS was added to the lower chamber of each chamber. Transfection was performed after 12 h, and the groups (3 wells per group) were divided as follows:
   blank control group: 20 uL of opti-MEM
   negative control group (NC group) and experimental group:
      A. 10 uL of opti-MEM and 0.2 uL of iMax were mixed well for 5 min
      B. 10 uL of opti-MEM and 0.5 uL of small RNA (final concentration 100 nmol/L) were mixed well
      C. solution A and solution B were mixed well and let stand for 15 min, and then was added to each well at a 20 uL system
   Note: 100 uL of nuclease-free water was added to 2 nmol of small RNA dry powder for dissolution at a storage concentration of 20 umol/L
2) Fixation, staining, photography and statistics:
   After 48 h, the medium in upper and lower chambers was discarded. The cells were fixed with paraformaldehyde at room temperature for 15 min, stained with 0.1% crystal violet at room temperature for 15 min, and rinsed with PBS. The cells in the upper chamber were gently wiped off with cotton swabs, and the chamber was dried. A Leica inverted microscope DMi8 was turned on, the 5× objective lens was selected, the parameters were adjusted and the white balance was performed following the manual. Each chamber was photographed, followed by counting and statistic processing. The results were as shown in Figure 14A and Figure 14B.

### Example 15. Verification of pro-apoptotic function of sRNA-163 derived from Maytenus hookerion H460 cells

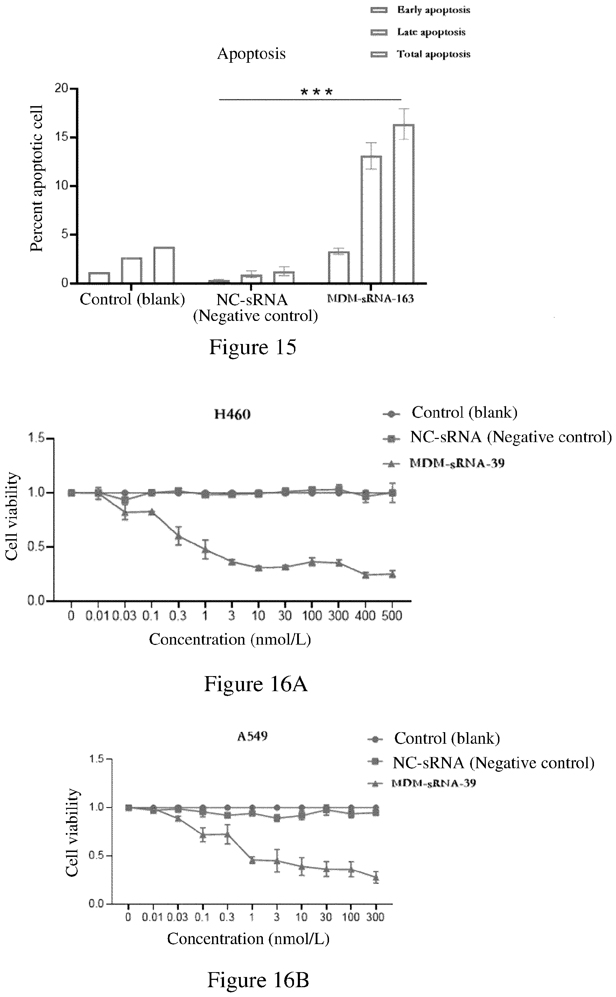

1) H460 cells with a cell density of more than 90% in 10 cm² dishes were plated in 12-well plates according to the cell growth rate, and transfected after 12 h, and the groups were divided as follows:
   blank control group (4 wells): 200 uL of opti-MEM per well
   negative control group (NC group) and experimental group (3 wells each): the system of each well was as follows
      A. 100 uL of opti-MEM and 2 uL of iMax were mixed well for 5 min
      B. 100 uL of opti-MEM and 5 uL of small RNA (final concentration 100 nmol/L) were mixed well
      C. solution A and solution B were mixed well and let stand for 15 min, and then was added to each well at a 200 uL system
   Note: 100 uL of nuclease-free water was added to 2 nmol of small RNA dry powder for dissolution at a storage concentration of 20 umol/L
2) Detection of apoptosis
   The supernatant and cells were collected after 48 h and washed twice with pre-chilled PBS. The 4 tubes of cells in the blank control group were added respectively with: (1) 200 uL of 1× binding buffer; (2) 200 uL of 1× binding buffer and 5 uL of Annexin V stain; (3) 200 uL of 1× binding buffer and 5 uL of PI stain; (4) 200 uL of 1× binding buffer, 5 uL of Annexin V stain and 5 uL of PI stain. 200 uL of 1× binding buffer, 5 uL of Annexin V stain and 5 uL of PI stain were added to each tube of both the NC group and the experimental group. The mixture was mixed well and filtered through 300 mesh filters. The live cell population and the position of quadrant gate were selected according to the blank control group, and then the apoptosis of the NC group and the experimental group was detected. The results were as shown in Figure 15.

### Example 16. Verification of concentration-dependent proliferation-inhibiting function of sRNA-39 derived from Maytenus hookeri on H460 and A549 cells

1) H460 and A549 cells with a cell density of more than 90% in 10 cm² dishes were plated in 96-well plates according to the cell growth rate, and transfected after 12 h, and the groups (3 wells per group) were divided as follows:
   blank control group: 20 uL of opti-MEM
   negative control group (NC group) and experimental group:
      A. 10 uL of opti-MEM and 2 uL of iMax were mixed well for 5 min
      B. 10 uL of opti-MEM and small RNA were mixed well (the concentration gradient of H460 cell experimental group was set as: 0.01 noml/L, 0.03 nmol/L, 0.1 noml/L, 0.3 noml/L, 1 noml/L, 3 noml/L, 10 noml/L, 30 noml/L, 100 noml/L, 300 noml/L, 400 noml/L, 500 noml/L; the concentration gradient of A549 cell experimental group was set as: 0.01 noml/L, 0.03 nmol/L, 0.1 noml/L, 0.3 noml/L, 1 noml/L, 3 noml/L, 10 noml/L, 30 noml/L, 100 noml/L, 300 noml/L; the final concentration of sRNA in NC group was 100 noml/L)
      C. solution A and solution B were mixed well and let stand for 15 min, and then was added to each well at a 20 uL system
   positive group: the concentration of paclitaxel was adjusted to 100 nmol/L
   Note: 100 uL of nuclease-free water was added to 2 nmol of small RNA dry powder for dissolution at a storage concentration of 20 umol/L
2) Detection of cell viability: MTS detection was performed after 48 h.

Formulation of MTS detection solution was as follows: opti-MEM: MTS A solution: MTS B solution = 100:20:1. After removing the supernatant, 100 uL of MTS detection solution was added to each well and incubated for 0.5-2 h. Absorbance values at wavelengths of 490 nm and 630 nm were measured in a microplate reader. The results were as shown in Figure 16A and Figure 16B.

### Example 17. Verification of concentration-dependent proliferation-inhibiting function of sRNA-163 derived from Maytenus hookeri on H460 and A549 cells

1) H460 and A549 cells with a cell density of more than 90% in 10 cm² dishes were plated in 96-well plates according to the cell growth rate, and transfected after 12 h, and the groups (3 wells per group) were divided as follows:
   blank control group: 20 uL of opti-MEM
   negative control group (NC group) and experimental group:
      A. 10 uL of opti-MEM and 0.2 uL of iMax were mixed well for 5 min
      B. 10 uL of opti-MEM and small RNA were mixed well (the concentration gradient of H460 cell experimental group was set as: 0.1 noml/L, 0.3 noml/L, 1 noml/L, 3 noml/L, 10 noml/L, 30 noml/L, 100 noml/L, 300 noml/L; the concentration gradient of A549 cell experimental group was set as: 0.01 noml/L, 0.03 nmol/L, 0.1 noml/L, 0.3 noml/L, 1 noml/L, 3 noml/L, 10 noml/L, 30 noml/L, 100 noml/L, 300 noml/L; the final concentration of sRNA in NC group was 100 noml/L)
      C. solution A and solution B were mixed well and let stand for 15 min, and then a 20 uL system was added at a 20 uL system
   Note: 100 uL of nuclease-free water was added to 2 nmol of small RNA dry powder for dissolution at a storage concentration of 20 umol/L
2) Detection of cell viability: MTS detection was performed after 48 h.

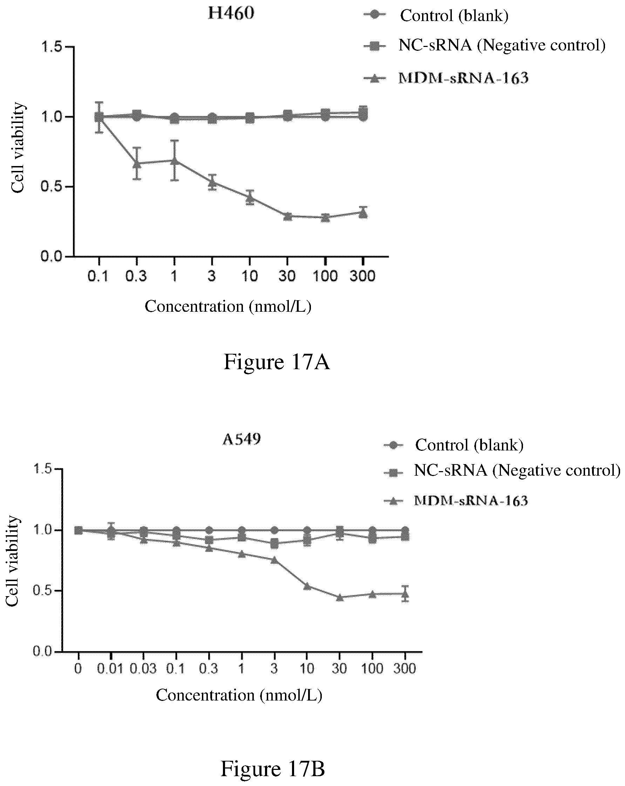

Formulation of MTS detection solution was as follows: opti-MEM: MTS A solution: MTS B solution = 100:20:1. After removing the supernatant, 100 uL of MTS detection solution was added to each well and incubated for 0.5-2 h. Absorbance values at wavelengths of 490 nm and 630 nm were measured in a microplate reader. The results were as shown in Figure 17A and Figure 17B.

### Example 18. MDM-sRNA-39 and MDM-sRNA-163 significantly reduced tumor burden of mice with primary lung cancer

The mouse model of primary lung cancer was Kras^{LSL-G12D}p53^{fl/fl} (KP).

Mice were weighed and assigned to the negative control group (NC group), MDM-sRNA-39 group and MDM-sRNA-163 group randomly. The NC group was the negative control, in which a sphingosine(d18:1)-NC-sRNA herbal medicine delivery system was constructed from NC-sRNA. The MDM-sRNA-39 group was an experimental group, in which a sphingosine(d18:1)-MDM-sRNA-39 herbal medicine delivery system was constructed from MDM-sRNA-39. The MDM-sRNA-163 group was an experimental group, in which a sphingosine(d18:1)-MDM-sRNA-163 herbal medicine delivery system was constructed from MDM-sRNA-163.

The herbal medicine delivery system was constructed by heating and mixing 10 nmol of sRNA, 100 ug of sphingosine(d18:1) and 300 ul of sterile enzyme-free water in a water bath at 90°C for 15 minutes.

Each mouse with a uniform body weight was dosed by gavage. Each mouse was given 10 nmol of HQi-sRNA-2 mimic daily by gavage for one month.

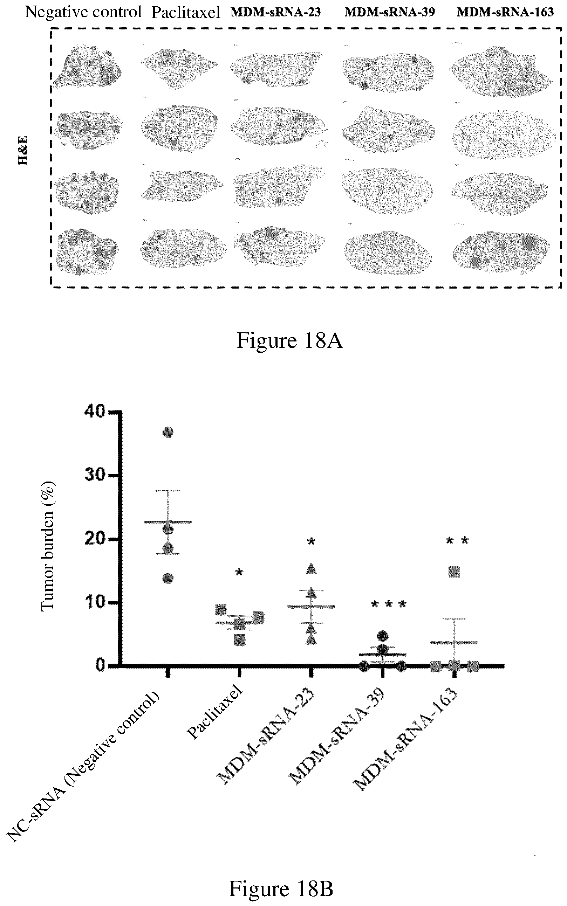

The lungs of mice were collected to prepare pathological sections. The left lung was collected and sliced at the largest surface for every mouse, and stained with HE. The area of tumor in the whole pathological section was counted, the tumor burden was calculated, and statistical analysis was performed. It was found that MDM-sRNA-39 and MDM-sRNA-163 significantly reduced the tumor burden of mice with primary lung cancer. The results were as shown in Figure 18A and Figure 18B.

### Example 19. MDM-sRNA-39 and MDM-sRNA-163 significantly prolonged the survival of mice with primary lung cancer

The mouse model of primary lung cancer was Kras^{LSL-G12D}p53^{fl/fl} (KP).

Mice were weighed and assigned to the negative control group (NC group), MDM-sRNA-39 group and MDM-sRNA-163 group randomly. The NC group was the negative control, in which a sphingosine(d18:1)-NC-sRNA herbal medicine delivery system was constructed from NC-sRNA. The MDM-sRNA-39 group was an experimental group, in which a sphingosine(d18:1)-MDM-sRNA-39 herbal medicine delivery system was constructed from MDM-sRNA-39. The MDM-sRNA-163 group was an experimental group, in which a sphingosine(d18:1)-MDM-sRNA-163 herbal medicine delivery system was constructed from MDM-sRNA-163.

The herbal medicine delivery system was constructed by heating and mixing 10 nmol of sRNA, 100 ug of sphingosine(d18:1) and 300 ul of sterile enzyme-free water in a water bath at 90°C for 15 minutes.

Each mouse with a uniform body weight was dosed by gavage. Each mouse was given 10 nmol of HQi-sRNA-2 mimic daily by gavage until death of the mouse.

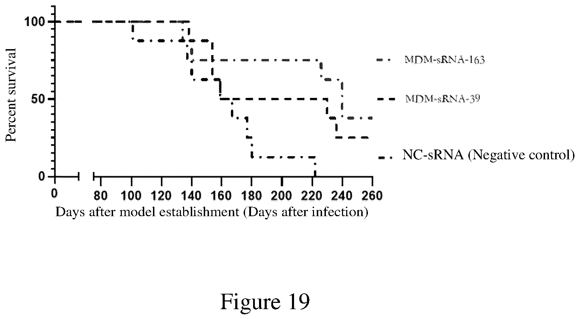

The survival time of each mouse was recorded, the survival curve was plotted, and statistical analysis was performed. It was found that MDM-sRNA-39 and MDM-sRNA-163 significantly prolonged the survival of mice with primary lung cancer. The results were as shown in Figure 19.

### Example 20. Concentration-dependent inhibition on activity of breast cancer MCF7 cells, breast cancer MDA-MB-231 cells, gastric cancer AGS cells, pancreatic cancer HS766T cells, pancreatic cancer ASPC-1 cells, and prostate cancer PC-3 cells by MDM-sRNA-39

1) Breast cancer MCF7 cells, breast cancer MDA-MB-231 cells, gastric cancer AGS cells, pancreatic cancer HS766T cells, pancreatic cancer ASPC-1 cells, and prostate cancer PC-3 cells with a cell density of more than 90% in 10 cm² plates were plated in 96-well according to the cell growth rate. Transfection was performed after 12 h, and the groups (3 wells per group) were divided as follows:
   blank control group: 20 uL of opti-MEM
   negative control group (NC group) and experimental group:
      A. 10 uL of opti-MEM and 0.2 uL of iMax were mixed well for 5 min
      B. 10 uL of opti-MEM and small RNA were mixed well (the concentration gradient of experimental group was set as: 0.01 noml/L, 0.03 noml/L, 0.1 noml/L, 0.39 noml/L, 0.78 noml/L, 1.56 noml/L, 3.125 noml/L, 6.25 noml/L, 12.5 noml/L, 25 noml/L, 50 noml/L, 100 noml/L; the final concentration of sRNA in NC group was 100 noml/L)
      C. solution A and solution B were mixed well and let stand for 15 min, and then was added at a 20 uL system
   Note: 100 uL of nuclease-free water was added to 2 nmol of small RNA dry powder for dissolution at a storage concentration of 20 umol/L
2) Detection of cell viability: MTS detection was performed after 48 h.

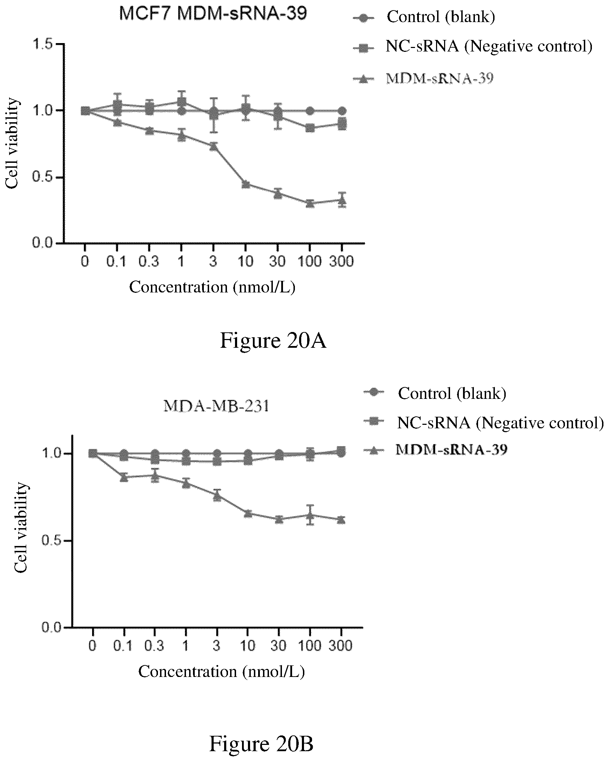

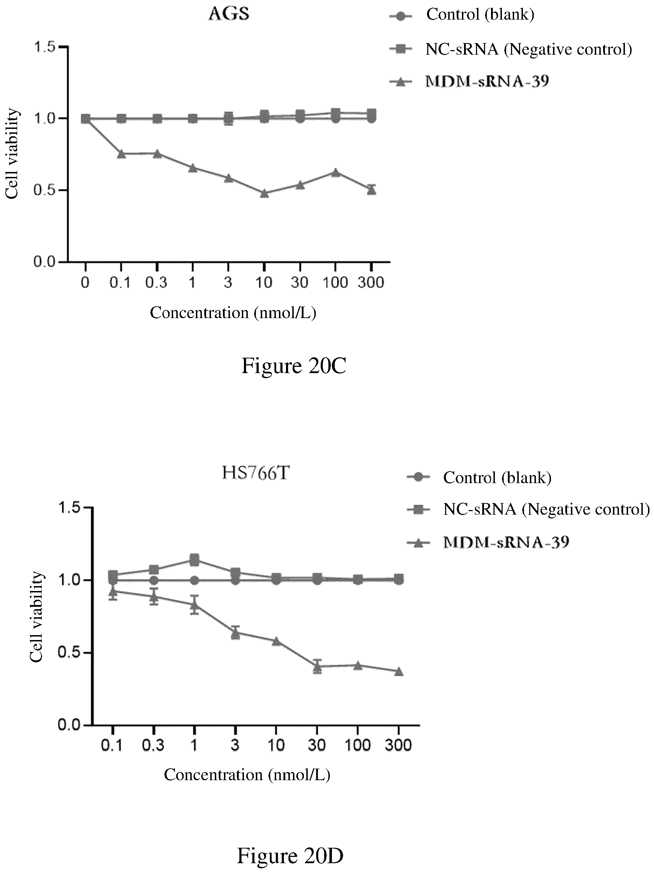

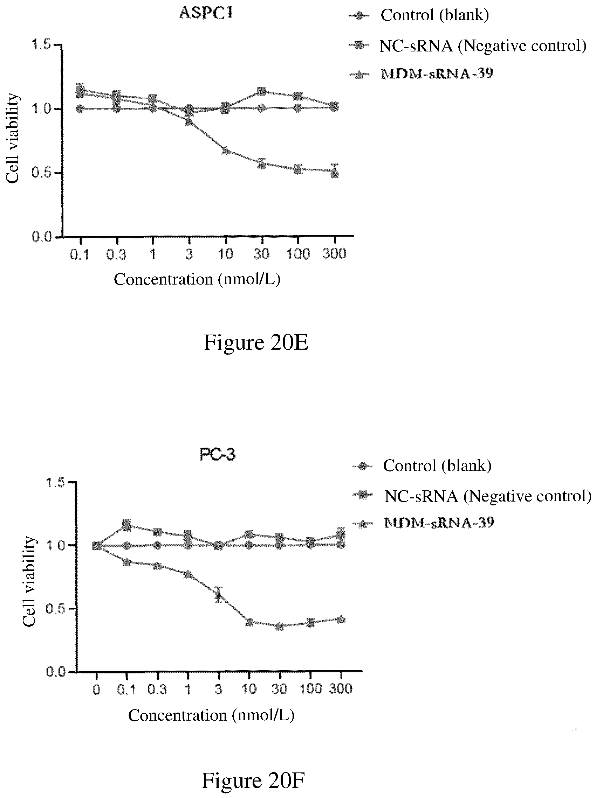

Formulation of MTS detection solution was as follows: opti-MEM: MTS A solution: MTS B solution = 100:20:1. After removing the supernatant, 100 uL of MTS detection solution was added to each well and incubated for 0.5-2 h. Absorbance values at wavelengths of 490 nm and 630 nm were measured in a microplate reader. It was found that the proliferation-inhibiting function of MDM-sRNA-39 on breast cancer MCF7 cells, breast cancer MDA-MB-231 cells, gastric cancer AGS cells, pancreatic cancer HS766T cells, pancreatic cancer ASPC-1 cells, and prostate cancer PC-3 cells was concentration-dependent. The results were as shown in Figure 20A to Figure 20F.

### Example 21. Concentration-dependent inhibition of the activity of breast cancer MCF7 cells, breast cancer MDA-MB-231 cells, gastric cancer AGS cells, pancreatic cancer HS766T cells, pancreatic cancer ASPC-1 cells, and prostate cancer PC-3 cells by MDM-sRNA-163

1) Breast cancer MCF7 cells, breast cancer MDA-MB-231 cells, gastric cancer AGS cells, pancreatic cancer HS766T cells, pancreatic cancer ASPC-1 cells, and prostate cancer PC-3 cells with a cell density of more than 90% in 10 cm² dishes were plated in 96-well plates according to the cell growth rate. Transfection was performed after 12 h, and the groups (3 wells per group) were divided as follows:
   blank control group: 20 uL of opti-MEM
   negative control group (NC group) and experimental group:
      A. 10 uL of opti-MEM and 0.2 uL of iMax were mixed well for 5 min
      B. 10 uL of opti-MEM and small RNA were mixed well (the concentration gradient of experimental group was set as: 0.01 noml/L, 0.03 noml/L, 0.1 noml/L, 0.39 noml/L, 0.78 noml/L, 1.56 noml/L, 3.125 noml/L, 6.25 noml/L, 12.5 noml/L, 25 noml/L, 50 noml/L, 100 noml/L; the final concentration of sRNA in NC group was 100 noml/L)
      C. solution A and solution B were mixed well and let stand for 15 min, and then was added at a 20 uL system
   Note: 100 uL of nuclease-free water was added to 2 nmol of small RNA dry powder for dissolution at a storage concentration of 20 umol/L
2) Detection of cell viability: MTS detection was performed after 48 h.

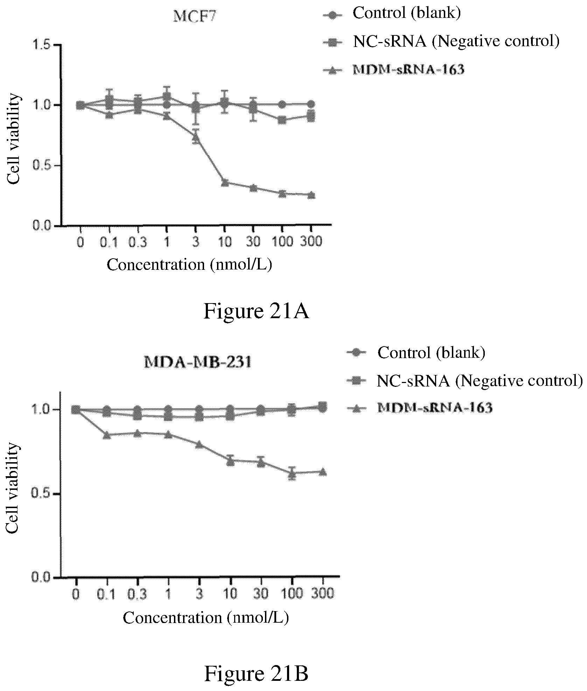

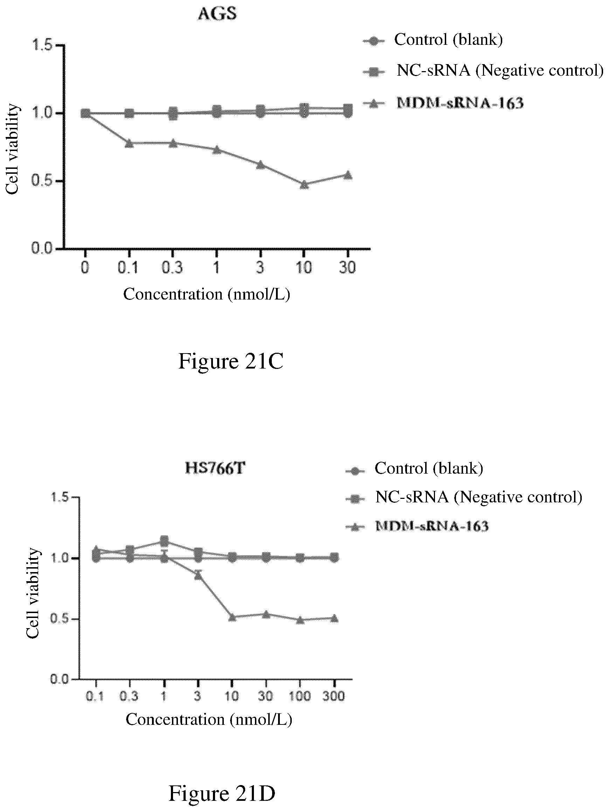

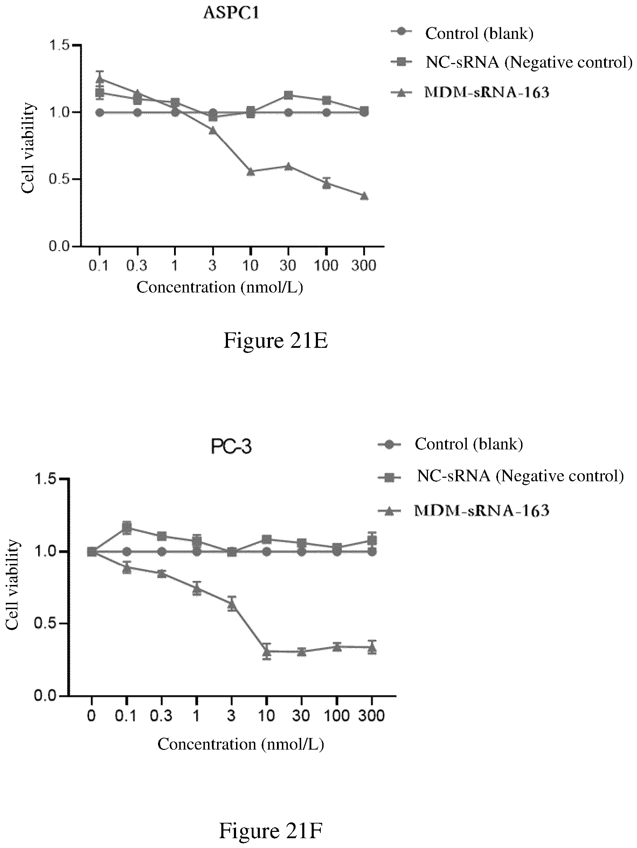

Formulation of MTS detection solution was as follows: opti-MEM: MTS A solution: MTS B solution = 100:20:1. After removing the supernatant, 100 uL of MTS detection solution was added to each well and incubated for 0.5-2 h. Absorbance values at wavelengths of 490 nm and 630 nm were measured in a microplate reader. It was found that the proliferation-inhibiting function of MDM-sRNA-163 on breast cancer MCF7 cells, breast cancer MDA-MB-231 cells, gastric cancer AGS cells, pancreatic cancer HS766T cells, pancreatic cancer ASPC-1 cells, and prostate cancer PC-3 cells was concentration-dependent. The results were as shown in Figure 21A to Figure 21F.

### Example 22. Verification of pro-apoptotic function of sRNA-39 derived from Maytenus hookeri on PANC-1 cells and BXPC-3 cells

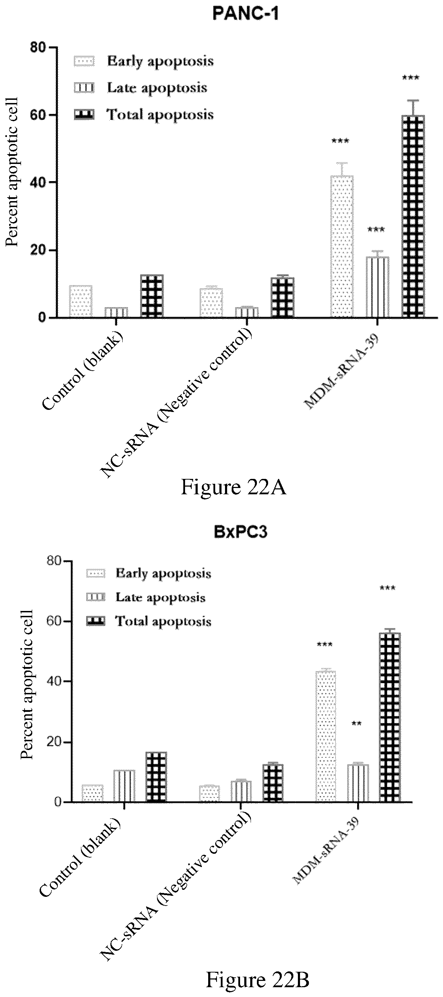

1) PANC-1 cells and BXPC-3 cells with a cell density of more than 90% in 10 cm² dishes were plated in 12-well plates according to the cell growth rate, and transfected after 12 h, and the groups were divided as follows:
   blank control group (4 wells): 100 uL of jetPRIME Buffer per well
   negative control group (NC group) and experimental group (3 wells each): 100 uL of jetPRIME Buffer was mixed well with 3 uL of jetPRIME reagent and 5 uL of small RNA for each well and let stand for 15 min, and then was added to each well at a 100 uL system (final concentration 100 nmol/L)
   Note: 100 uL of nuclease-free water was added to 2 nmol of small RNA dry powder for dissolution at a storage concentration of 20 umol/L
2) Detection of apoptosis:
   The supernatant and cells were collected after 72 h and washed twice with pre-chilled PBS. The 4 tubes of cells in the blank control group were added respectively with: (1) 200 uL of 1× binding buffer; (2) 200 uL of 1× binding buffer and 5 uL of Annexin V stain; (3) 200 uL of 1× binding buffer and 5 uL of PI stain; (4) 200 uL of 1× binding buffer, 5 uL of Annexin V stain and 5 uL of PI stain. 200 uL of 1× binding buffer, 5 uL of Annexin V stain and 5 uL of PI stain were added to each tube of both the NC group and the experimental group. The mixture was mixed well and filtered through 300 mesh filters. The live cell population and the position of quadrant gate were selected according to the blank control group, and then the apoptosis of the NC group and the experimental group was detected. The results were as shown in Figure 22A to Figure 22B.

### Example 23. Verification of migration-inhibiting function of sRNA-39 derived from Maytenus hookeri on ASPC-1 cells and BXPC-3 cells

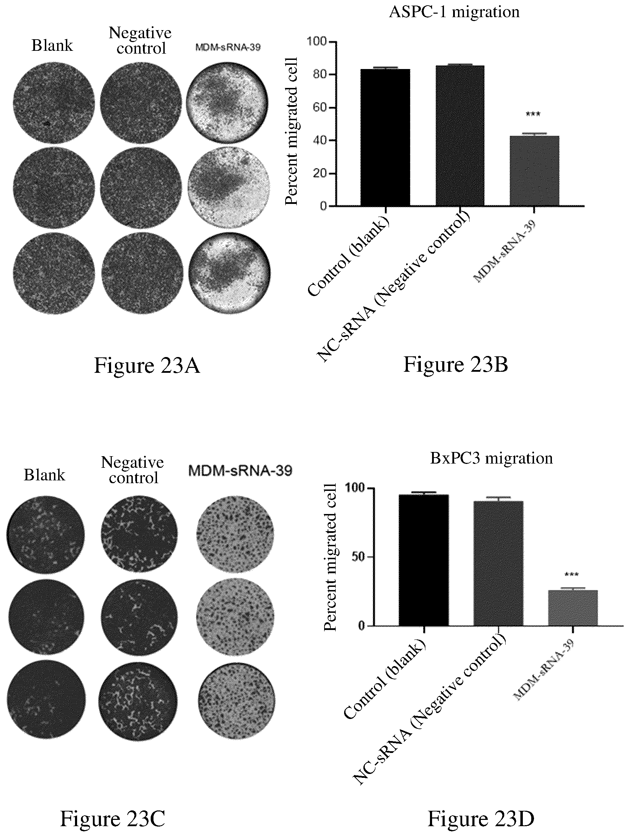

1) ASPC-1 cells and BXPC-3 cells with a cell density of more than 90% in 10 cm² dishes were digested and then resuspended with 1 mL of serum-free medium and counted. 100,000 cells (100 uL) were plated in the upper chamber, and complete medium with 10% FBS was added to the lower chamber of each Transwell chamber. Transfection was performed after 12 h, and the groups (3 wells per group) were divided:
   blank control group: 10 uL of jetPRIME Buffer
   negative control group (NC group) and experimental group: 10 uL of jetPRIME Buffer was mixed well with 0.5 uL of jetPRIME reagent and 0.5 uL of small RNA and let stand for 15 min, and then was added to each well at a 10 uL system (final concentration 100 nmol/L)
   Note: 100 uL of nuclease-free water was added to 2 nmol of small RNA dry powder for dissolution at a storage concentration of 20 umol/L
2) Fixation, staining, photography and statistics:
   After 72 h, the medium in upper and lower chambers was discarded. The cells were fixed with paraformaldehyde at room temperature for 15 min, stained with 0.1% crystal violet at room temperature for 15 min, and rinsed with PBS. The cells in the upper chamber were gently wiped off with cotton swabs, and the chamber was dried. A Leica inverted microscope DMi8 was turned on, the 5× objective lens was selected, the parameters were adjusted and the white balance was performed following the manual. Each chamber was photographed, followed by counting and statistic processing. The results were as shown in Figure 23A to Figure 23D.

### Example 24. Verification of invasion-inhibiting function of sRNA-39 derived from Maytenus hookeri on ASPC-1 cells and BXPC-3 cells

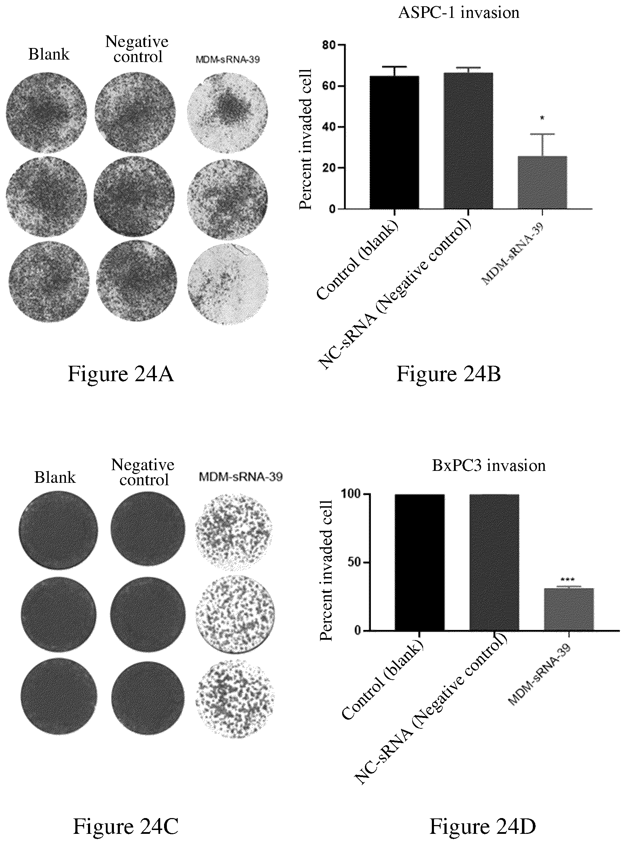

1) Matrigel basement membrane matrix was thawed on crushed ice one night before and diluted with serum-free medium to a final concentration of 200-300 ug/mL. 100 uL of the diluted Matrigel was added to each upper chamber, let stand at 37°C for 30 min, and the supernatant was aspirated. ASPC-1 cells and BXPC-3 cells with a cell density of more than 90% in 10 cm² dishes were digested and resuspended with 1 mL of serum-free medium and counted. 100,000 cells (100 uL) were plated in the upper chamber, and complete medium with 10% FBS was added to the lower chamber of each chamber. Transfection was performed after 12 h, and the groups (3 wells per group) were divided as follows:
   blank control group: 10 uL of jetPRIME Buffer
   negative control group (NC group) and experimental group: 10 uL of jetPRIME Buffer was mixed well with 0.5 uL of jetPRIME reagent and 0.5 uL of small RNA and let stand for 15 min, and then was added to each well at a 10 uL system (final concentration 100 nmol/L)
   Note: 100 uL of nuclease-free water was added to 2 nmol of small RNA dry powder for dissolution at a storage concentration of 20 umol/L
2) Fixation, staining, photography and statistics:
   After 72 h, the medium in upper and lower chambers was discarded. The cells were fixed with paraformaldehyde at room temperature for 15 min, stained with 0.1% crystal violet at room temperature for 15 min, and rinsed with PBS. The cells in the upper chamber were gently wiped off with cotton swabs, and the chamber was dried. A Leica inverted microscope DMi8 was turned on, the 5× objective lens was selected, the parameters were adjusted and the white balance was performed following the manual. Each chamber was photographed, followed by counting and statistic processing. The results were as shown in Figure 24A to Figure 24D.

### Example 25. Verification of pro-apoptotic function of sRNA-163 derived from Maytenus hookeri on PANC-1 cells and BXPC-3 cells

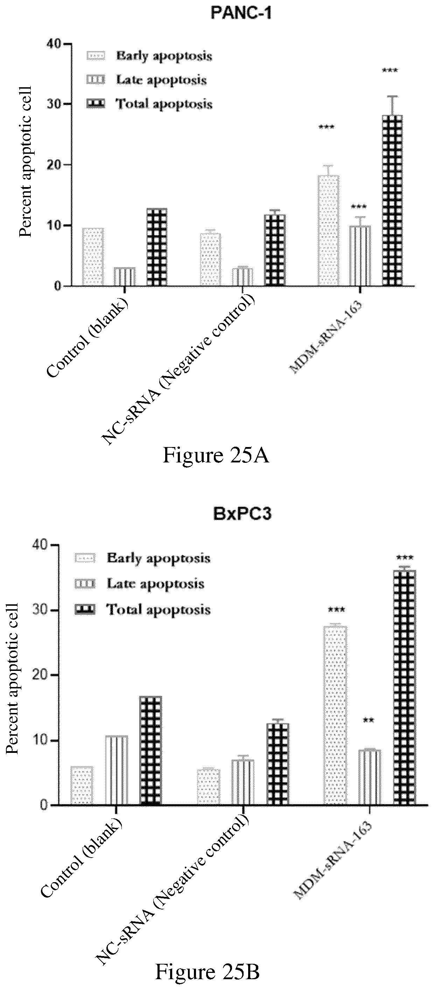

1) PANC-1 cells and BXPC-3 cells with a cell density of more than 90% in 10 cm² dishes were plated in 12-well plates according to the cell growth rate, and transfected after 12 h, and the groups were divided as follows:
   blank control group (4 wells): 100 uL of jetPRIME Buffer per well
   negative control group (NC group) and experimental group (3 wells each): 100 uL of jetPRIME Buffer was mixed with 3 uL of j etPRIME reagent and 5 uL of small RNA per well and let stand for 15 min, and then was added to each well at a 100 uL system (final concentration 100 nmol/L)
   Note: 100 uL of nuclease-free water was added to 2 nmol of small RNA dry powder for dissolution at a storage concentration of 20 umol/L
2) Detection of apoptosis:
   The supernatant and cells were collected after 72 h amd washed twice with pre-chilled PBS. The 4 tubes of cells in the blank control group were added respectively with: (1) 200 uL of 1× binding buffer; (2) 200 uL of 1× binding buffer and 5 uL of Annexin V stain; (3) 200 uL of 1× binding buffer and 5 uL of PI stain; (4) 200 uL of 1× binding buffer, 5 uL of Annexin V stain and 5 uL of PI stain. 200 uL of 1× binding buffer, 5 uL of Annexin V stain and 5 uL of PI stain were added to each tube of both the NC group and the experimental group. The mixture was mixed well and filtered through 300 mesh filters. The live cell population and the position of quadrant gate were selected according to the blank control group, and then the apoptosis of the NC group and the experimental group was detected. The results were as shown in Figure 25A to Figure 25B.

### Example 26. Verification of migration-inhibiting function of sRNA-163 derived from Maytenus hookeri on ASPC-1 cells and BXPC-3 cells

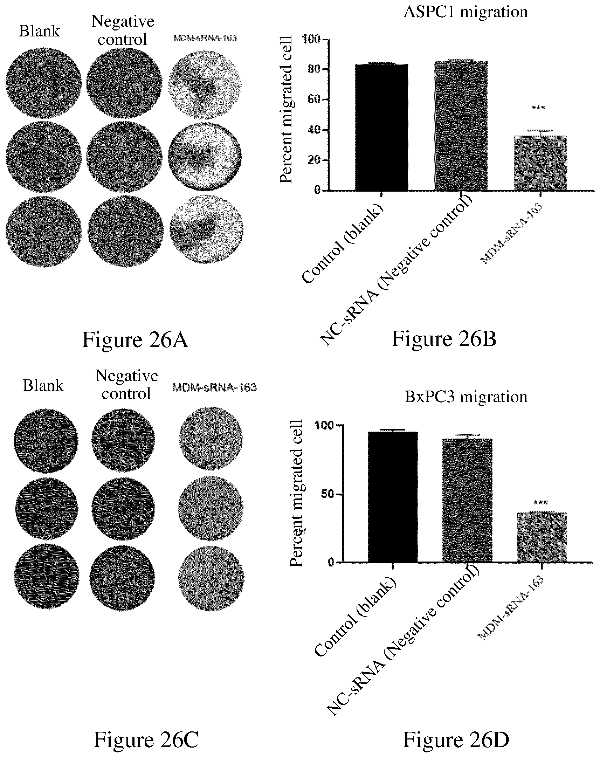

1) ASPC-1 cells and BXPC-3 cells with a cell density of more than 90% in 10 cm² dishes were digested and then resuspended with 1 mL of serum-free medium and counted. 100,000 cells (100 uL) were plated in the upper chamber, and complete medium with 10% FBS was added to the lower chamber of each Transwell chamber. Transfection was performed after 12 h, and the groups (3 wells per group) were divided as follows:
   blank control group: 10 uL of jetPRIME Buffer
      negative control group (NC group) and experimental group: 10 uL of jetPRIME Buffer was mixed well with 0.5 uL of j etPRIME reagent and 0.5 uL of small RNA and let stand for 15 min, and then was added to each well at a 10 uL system (final concentration 100 nmol/L)
   Note: 100 uL of nuclease-free water was added to 2 nmol of small RNA dry powder for dissolution at a storage concentration of 20 umol/L
2) Fixation, staining, photography and statistics:
   After 72 h, the medium in upper and lower chambers was discarded. The cells were fixed with paraformaldehyde at room temperature for 15 min, stained with 0.1% crystal violet at room temperature for 15 min, and rinsed with PBS. The cells in the upper chamber were gently wiped off with cotton swabs, and the chamber was dried. A Leica inverted microscope DMi8 was turned on, the 5× objective lens was selected, the parameters were adjusted and the white balance was performed following the manual. Each chamber was photographed, followed by counting and statistic processing. The results were as shown in Figure 26A to Figure 26D.

### Example 27. MDM-sRNA-163 inhibited invasion of pancreatic cancer BXPC-3 cells

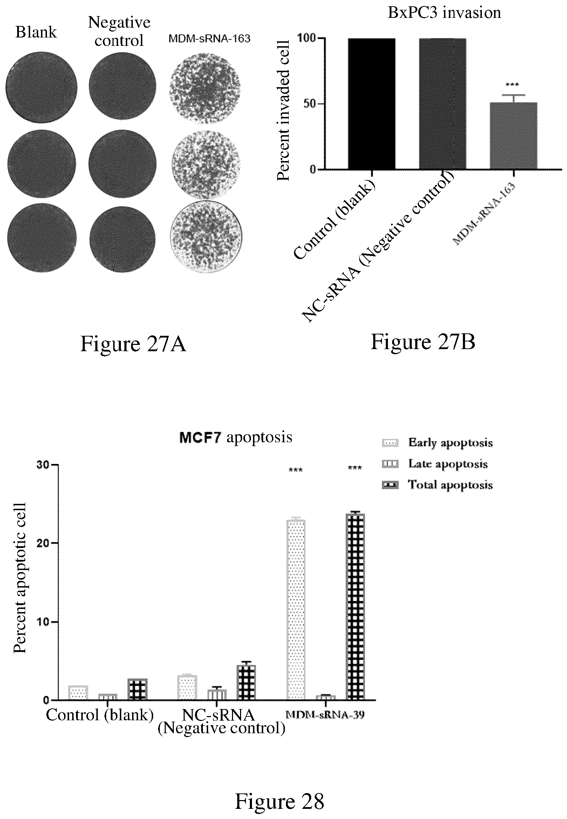

1) Matrigel basement membrane matrix was thawed on crushed ice one night before and diluted with serum-free medium to a final concentration of 200-300 ug/mL. 100 uL of the diluted Matrigel was added to each upper chamber, let stand at 37°C for 30 min, and the supernatant was aspirated. BXPC-3 cells with a cell density of more than 90% in 10 cm² dishes were digested and resuspended with 1 mL of serum-free medium and counted. 100,000 cells (100 uL) were plated in the upper chamber, and complete medium with 10% FBS was added to the lower chamber of each chamber. Transfection was performed after 12 h, and the groups (3 wells per group) were divided as follows:
   blank control group: 10 uL of jetPRIME Buffer
   NC group and experimental group: 10 uL of jetPRIME Buffer was mixed well with 0.5 uL of jetPRIME reagent and 0.5 uL of small RNA and let stand for 15 min, and then was added to each well at a 10 uL system (final concentration 100nmol/L)
   Note: 100 uL of nuclease-free water was added to 2 nmol of small RNA dry powder for dissolution at a storage concentration of 20 umol/L
2) Fixation, staining, photography and statistics:
   After 72 h, the medium in upper and lower chambers was discarded. The cells were fixed with paraformaldehyde at room temperature for 15 min, stained with 0.1% crystal violet at room temperature for 15 min, and rinsed with PBS. The cells in the upper chamber were gently wiped off with cotton swabs, and the chamber was dried. A Leica inverted microscope DMi8 was turned on, the 5× objective lens was selected, the parameters were adjusted and the white balance was performed following the manual. Each chamber was photographed, followed by counting and statistic processing. The results were as shown in Figure 27A to Figure 27B.

### Example 28. MDM-sRNA-39 promoted apoptosis of breast cancer MCF7 cells

1) MCF-7 cells with a cell density of more than 90% in 10 cm² dishes were plated in 12-well plates according to the cell growth rate, and transfected after 12 h, and the groups were divided as follows:
   blank control group (4 wells): 200 uL of opti-MEM per well
   negative control group (NC group) and experimental group (3 wells each):
      A. 100 uL of opti-MEM and 2 uL of iMax were mixed well for 5 min
      B. 100 uL of opti-MEM and 5 uL of small RNA (final concentration 100 nmol/L) were mixed well
      C. solution A and solution B were mixed well and let stand for 15 min, and then was added to each well at a 200 uL system
   Note: 100 uL of nuclease-free water was added to 2 nmol of small RNA dry powder for dissolution at a storage concentration of 20 umol/L
2) Detection of apoptosis:
   The supernatant and cells were collected after 48 h and washed twice with pre-chilled PBS. The 4 tubes of cells in the blank control group were added respectively with: (1) 200 uL of 1 × binding buffer; (2) 200 uL of 1 × binding buffer and 5 uL of Annexin V stain; (3) 200 uL of 1 × binding buffer and 5 uL of PI stain; (4) 200 uL of 1× binding buffer, 5 uL of Annexin V stain and 5 uL of PI stain. 200 uL of 1× binding buffer, 5 uL of Annexin V stain and 5 uL of PI stain were added to each tube of both the NC group and the experimental group. The mixture was mixed well and filtered through 300 mesh filters. The live cell population and the position of quadrant gate were selected according to the blank control group, and then the apoptosis of the NC group and the experimental group was detected. The results were as shown in Figure 28.

### Example 29. MDM-sRNA-39 promoted apoptosis of prostate cancer PC-3 cells

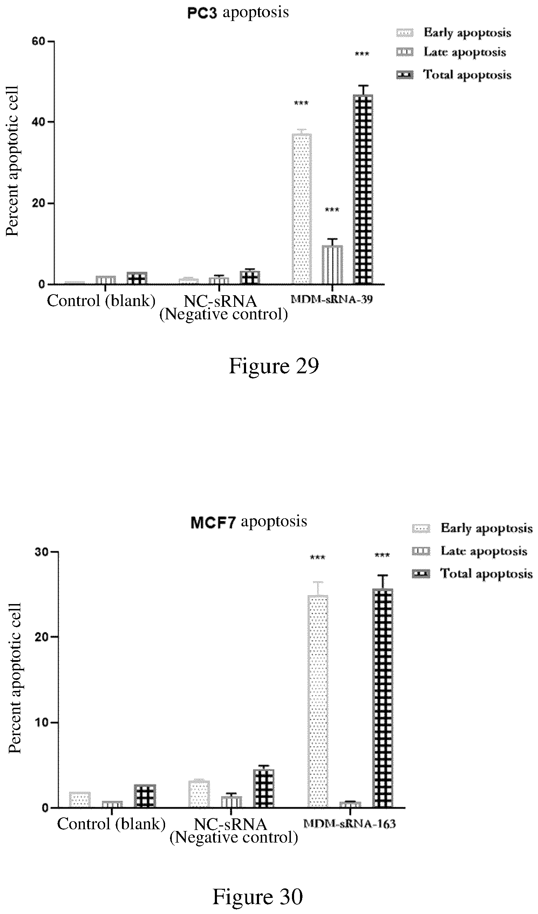

1) PC-3 cells with a cell density of more than 90% in 10 cm² dishes were plated in 12-well plates according to the cell growth rate, and transfected after 12 h, and the groups were divided as follows:
   blank control group (4 wells): 200 uL of opti-MEM per well
   negative control group (NC group) and experimental group (3 wells each): the system of each well was as follows
      A. 100 uL of opti-MEM and 2 uL of iMax were mixed well for 5 min
      B. 100 uL of opti-MEM and 5 uL of small RNA (final concentration 100 nmol/L) were mixed well
      C. solution A and solution B were mixed well and let stand for 15 min, and then was added to each well at a 200 uL system
   Note: 100 uL of nuclease-free water was added to 2 nmol of small RNA dry powder for dissolution at a storage concentration of 20 umol/L
2) Detection of apoptosis:
   The supernatant and cells were collected after 48 h and washed twice with pre-chilled PBS. The 4 tubes of cells in the blank control group were added respectively with: (1) 200 uL of 1 × binding buffer; (2) 200 uL of 1 × binding buffer and 5 uL of Annexin V stain; (3) 200 uL of 1 × binding buffer and 5 uL of PI stain; (4) 200 uL of 1× binding buffer, 5 uL of Annexin V stain and 5 uL of PI stain. 200 uL of 1× binding buffer, 5 uL of Annexin V stain and 5 uL of PI stain were added to each tube of both the NC group and the experimental group. The mixture was mixed well and filtered through 300 mesh filters. The live cell population and the position of quadrant gate were selected according to the blank control group, and then the apoptosis of the NC group and the experimental group was detected. The results were as shown in Figure 29.

### Example 30. MDM-sRNA-163 promoted apoptosis of breast cancer MCF7 cells

1) MCF7 cells with a cell density of more than 90% in 10 cm² dishes were plated in 12-well plates according to the cell growth rate, and transfected after 12 h, and the groups were divided as follows:
   blank control group (4 wells): 200 uLof opti-MEM per well
   negative control group (NC group) and experimental group (3 wells each): the system of each well was as follows
      A. 100 uL of opti-MEM and 2 uL of iMax were mixed well for 5 min
      B. 100 uL of opti-MEM and 5 ul of small RNA (final concentration 100 nmol/L) were mixed well
      C. solution A and solution B were mixed well and let stand for 15 min, and then was added to each well at a 200 uL system
   Note: 100 uL of nuclease-free water was added to 2 nmol of small RNA dry powder for dissolution at a storage concentration of 20 umol/L
2) Detection of apoptosis:
   The supernatant and cells were collected after 48 h and washed twice with pre-chilled PBS. The 4 tubes of cells in the blank control group were added respectively with: (1) 200 uL of 1 × binding buffer; (2) 200 uL of 1 × binding buffer and 5 uL of Annexin V stain; (3) 200 uL of 1 × binding buffer and 5 uL of PI stain; (4) 200 uL of 1× binding buffer, 5 uL of Annexin V stain and 5 uL of PI stain. 200 uL of 1× binding buffer, 5 uL of Annexin V stain and 5 uL of PI stain were added to each tube of both the NC group and the experimental group. The mixture was mixed well and filtered through 300 mesh filters. The live cell population and the position of quadrant gate were selected according to the blank control group, and then the apoptosis of the NC group and the experimental group was detected. The results were as shown in Figure 30.

### Example 31. MDM-sRNA-163 promoted apoptosis of prostate cancer PC-3 cells

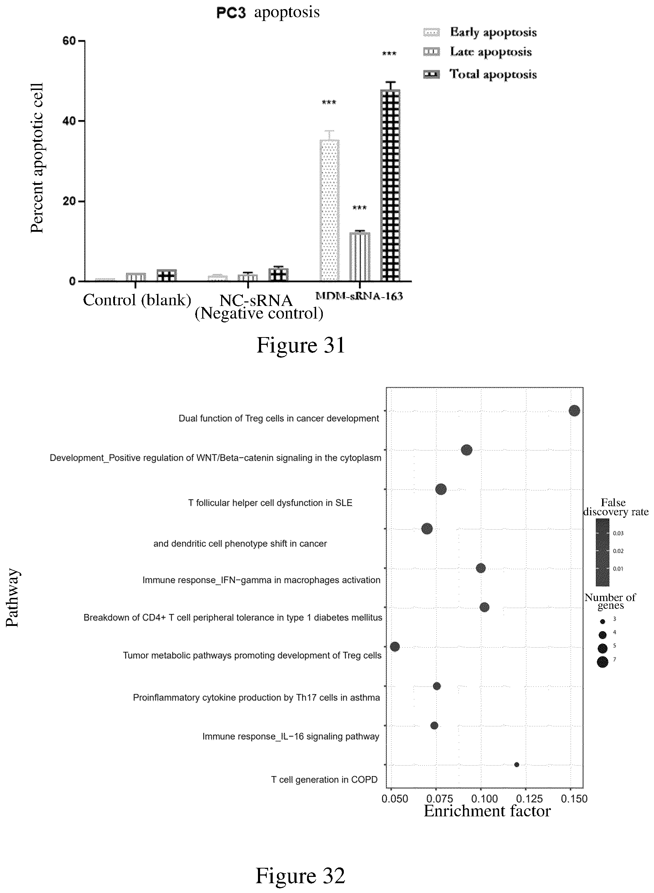

1) PC-3 cells with a cell density of more than 90% in 10 cm² dishes were plated in 12-well plates according to the cell growth rate, and transfected after 12 h, and the groups were divided as follows:
   blank control group (4 wells): 200 uL of opti-MEM per well
   negative control group (NC group) and experimental group (3 wells each): the system of each well was as follows
      A. 100 uL of opti-MEM and 2 uL of iMax were mixed well for 5 min
      B. 100 uL of opti-MEM and 5 uL of small RNA (final concentration 100 nmol/L) were mixed well
      C. solution A and solution B were mixed well and let stand for 15 min, and then was added to each well at a 200 uL system
   Note: 100 uL of nuclease-free water was added to 2 nmol of small RNA dry powder for dissolution at a storage concentration of 20 umol/L
2) Detection of apoptosis:
   The supernatant and cells were collected after 48 h and washed twice with pre-chilled PBS. The 4 tubes of cells in the blank control group were added respectively with: (1) 200 uL of 1 × binding buffer; (2) 200 uL of 1 × binding buffer and 5 uL of Annexin V stain; (3) 200 uL of 1 × binding buffer and 5 uL of PI stain; (4) 200 uL of 1× binding buffer, 5 uL of Annexin V stain and 5 uL of PI stain. 200 uL of 1× binding buffer, 5 uL of Annexin V stain and 5 uL of PI stain were added to each tube of both the NC group and the experimental group. The mixture was mixed well and filtered through 300 mesh filters. The live cell population and the position of quadrant gate were selected according to the blank control group, and then the apoptosis of the NC group and the experimental group was detected. The results were as shown in Figure 31.

### Example 32. Functions of genes down-regulated at transcriptional level by HQi-sRNA-2 mainly clustered in oncology, immunology and other related biological pathways

The mouse model of primary lung cancer was Kras^{LSL-G12D}p53^{fl/fl} (KP). Mice were weighed and assigned to the NC group and HQi-sRNA-2 group randomly. The NC group was the negative control, in which a sphingosine(d18:1)-NC-sRNA herbal medicine delivery system was constructed from NC-sRNA. The HQi-sRNA-2 group was the experimental group, in which a sphingosine(d18:1)-HQi-sRNA-2 herbal medicine delivery system was constructed from HQi-sRNA-2. The herbal medicine delivery system was constructed by heating and mixing 10 nmol of sRNA, 100 ug of sphingosine(d18:1) and 300 ul of sterile enzyme-free water in a water bath at 90°C for 15 minutes. Mouse lung tissue was collected after 30 days of dosing, and the total RNA was extracted and subjected to non-strand-specific transcriptome sequencing. The differentially expressed genes in the experimental group and the control group were analyzed and calculated by a bioinformatics software, and the biological pathways in which the differentially expressed genes mainly functioned were analyzed by clustering using the metacore database. The pathways of the differentially expressed genes were visualized by the visualization software package ggplot2. The results were as shown in Figure 32.

### Example 33. Functions of genes down-regulated at transcriptional level by MDM-sRNA-39 mainly clustered in oncology, immunology and other related biological pathways

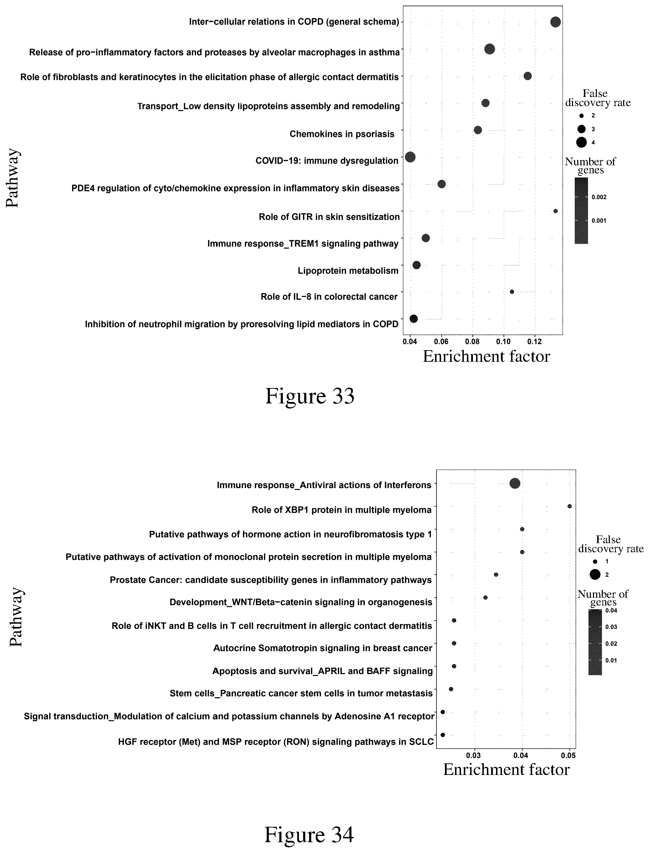

The mouse model of primary lung cancer was Kras^{LSL-G12D}p53^{fl/fl} (KP). Mice were weighed and assigned to the NC group and MDM-sRNA-39 group randomly. The NC group was the negative control, in which a sphingosine(d18:1)-NC-sRNA herbal medicine delivery system was constructed from NC-sRNA. The MDM-sRNA-39 group was the experimental group, in which a sphingosine(d18:1)-MDM-sRNA-39 herbal medicine delivery system was constructed from MDM-sRNA-39. The herbal medicine delivery system was constructed by heating and mixing 10 nmol of sRNA, 100 ug of sphingosine(d18:1) and 300 ul of sterile enzyme-free water in a water bath at 90°C for 15 minutes. Mouse lung tissue was collected after 30 days of dosing, and the total RNA was extracted and subjected to non-strand-specific transcriptome sequencing. The differentially expressed genes in the experimental group and the control group were analyzed and calculated by a bioinformatics software, and the biological pathways in which the differentially expressed genes mainly functioned were analyzed by clustering using the metacore database. The pathways of the differentially expressed genes were visualized by the visualization software package ggplot2. The results were as shown in Figure 33.

### Example 34. Functions of genes down-regulated at transcriptional level by MDM-sRNA-163 mainly clustered in oncology, immunology and other related biological pathways

The mouse model of primary lung cancer was Kras^{LSL-G12D}p53^{fl/fl} (KP). Mice were weighed and assigned to the NC group and MDM-sRNA-163 group randomly. The NC group was the negative control, in which a sphingosine(d18:1)-NC-sRNA herbal medicine delivery system was constructed from NC-sRNA. The MDM-sRNA-163 group was the experimental group, in which a sphingosine(d18:1)-MDM-sRNA-163 herbal medicine delivery system was constructed from MDM-sRNA-163. The herbal medicine delivery system was constructed by heating and mixing 10 nmol of sRNA, 100 ug of sphingosine(d18:1) and 300 ul of sterile enzyme-free water in a water bath at 90°C for 15 minutes. Mouse lung tissue was collected after 30 days of dosing, and the total RNA was extracted and subjected to non-strand-specific transcriptome sequencing. The differentially expressed genes in the experimental group and the control group were analyzed and calculated by a bioinformatics software, and the biological pathways in which the differentially expressed genes mainly functioned were analyzed by clustering using the metacore database. The pathways of the differentially expressed genes were visualized by the visualization software package ggplot2. The results were as shown in Figure 34.

### Example 35. HQi-sRNA-2 could significantly down-regulate the mRNA and protein levels of cyclooxygenase-2 (COX2) in H460 cells and lung tissues of mice with lung cancer

Method for cell experiment: H460 cells with a cell density of more than 90% in 10 cm2 dishes were plated in 12-well plates (2 plates) according to the cell growth rate, and transfected after 12 h, and the groups were divided as follows:
blank control group (4 wells): 200 uL of opti-MEM per well
negative control group (NC group) and experimental group (3 wells each): the system of each well was as follows
   A. 100 uL of opti-MEM and 2 uL of iMax were mixed well for 5 min
   B. 100 uL of opti-MEM and 5 uL of small RNA (final concentration 10 nmol/L) were mixed well
   C. solution A and solution B were mixed well and let stand for 15 min, and then was added to each well at a 200 uL system
Note: 100 uL of nuclease-free water was added to 2 nmol of small RNA dry powder for dissolution at a storage concentration of 20 umol/L

Cells were collected with Trizo after 24 h of incubation and RNA was extracted. Cells on another plate were collected with RIPA lysis buffer after 48 h of incubation and protein was extracted.

Methods for animal experiment: the mouse model of primary lung cancer was KrasLSL-G12Dp53fl/fl (KP).

Mice were weighed and assigned to the NC group and HQi-sRNA-2 group randomly. The NC group was the negative control, in which a sphingosine(d18:1)-NC-sRNA herbal medicine delivery system was constructed from NC-sRNA. The HQi-sRNA-2 group was the experimental group, in which a sphingosine(d18:1)-HQi-sRNA-2 herbal medicine delivery system was constructed from HQi-sRNA-2.

The herbal medicine delivery system was constructed by heating and mixing 10 nmol of sRNA, 100 ug of sphingosine(d18:1) and 300 ul of sterile enzyme-free water in a water bath at 90°C for 15 minutes.

Each mouse with a uniform body weight was dosed by gavage. Each mouse was given 10 nmol of HQi-sRNA-2 mimic daily by gavage for one month.

RNA and protein were extracted from the right lung of all mice.

The relative amount of COX-2 mRNA in the RNA in Method 1 and Method 2 was measured by RT-PCR and QPCR technologies. It was found that HQi-sRNA-2 could significantly down-regulate the mRNA level of COX2 in H460 cells and lung tissues of mice with lung cancer.

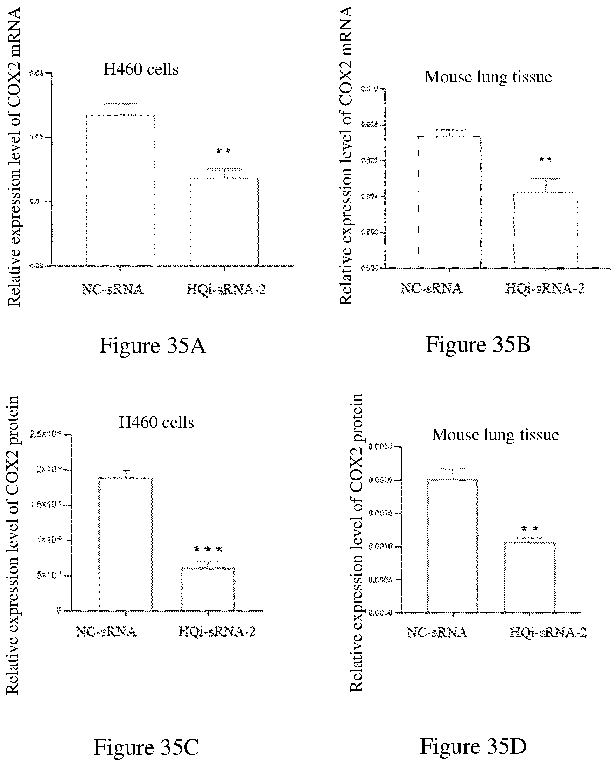

The relative amount of COX-2 protein in the protein in Method 1 and Method 2 was measured by western blot technology. It was found that HQi-sRNA-2 could significantly down-regulate the protein level of COX2 in H460 cells and lung tissues of mice with lung cancer. The results were as shown in Figure 35A to Figure 35D.

### Example 36. Luciferase reporter assay demonstrated that HQi-sRNA-2 could down-regulate cyclooxygenase-2 (COX2) expression, confirming that COX2 was a target of HQi-sRNA-2

The targeting effect of HQi-sRNA-2 obtained from medicinal plants by the inventors on cyclooxygenase-2 (COX2) was studied by a dual luciferase reporter detection system, so as to study its potential to exert lipid-lowering effects.

293T cells were cultured and transfected with the HQi-sRNA-2 to be screened and NC-sRNA, respectively, and then with the dual luciferase reporter plasmid of COX2. The dual luciferase activity was detected after 48 h.

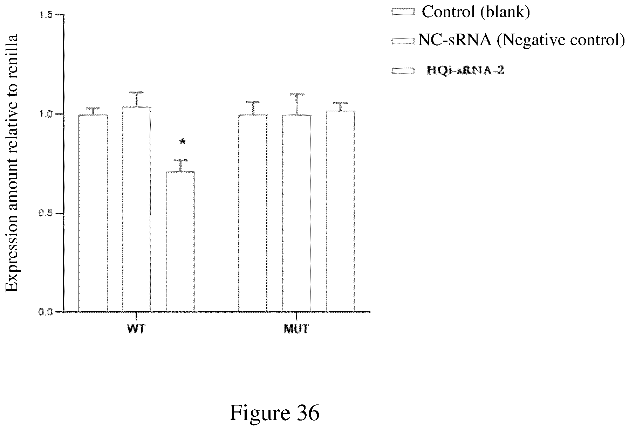

HQi-sRNA-2 could significantly reduce the luciferase activity by base pairing with the COX2 transcript sequence compared with the negative control group (NC-sRNA). Mutation of the base sequence of the binding site restored the luciferase activity, demonstrating that HQi-sRNA-2 may down-regulate the expression of COX2 gene by base pairing with the COX2 transcript sequence. The results were as shown in Figure 36.

### Note:

H460 human large cell lung cancer cells
H23 human non-small cell lung cancer cells
A549 human non-small cell lung cancer cells
PC3 human prostate cancer cells
MCF7 human breast cancer cells
HS766T human pancreatic cancer cells
ASPC-1 human metastatic pancreatic adenocarcinoma cells
AGS human gastric adenocarcinoma cells
PANC1 human breast cancer cells
BXPC3 human pancreatic adenocarcinoma *in situ* cells

## Claims

1. An isolated nucleic acid molecule comprising or consisting of the following sequence:
A) a nucleotide sequence as shown in any one of SEQ ID NO: 1 to SEQ ID NO: 133;
B) a sequence having at least 70%, at least 75%, at least 80%, at least 85%, at least 90%, at least 95%, 96%, 97%, 98% or 99% sequence identity with a nucleotide sequence as shown in any one of SEQ ID NO: 1 to SEQ ID NO: 133;
C) a sequence having up to 10 nucleotide substitutions, deletions, or additions compared with a nucleotide sequence as shown in any one of SEQ ID NO: 1 to SEQ ID NO: 133, preferably a sequence having 9, 8, 7, 6, 5, 4, 3, 2 or 1 nucleotide substitution, deletion, or addition,
D) a sequence capable of hybridizing to a nucleotide sequence as shown in any one of SEQ ID NO: 1 to SEQ ID NO: 133 under stringent conditions, or
E) a nucleotide sequence comprising any one of the core sequences listed in Table 4.

2. The isolated nucleic acid molecule according to claim 1, which is an RNA molecule or a DNA molecule, preferably a small RNA molecule, preferably a small RNA molecule with a length of 18-36 nucleotides, preferably a small RNA molecule with a length of 18, 19, 20, 21, 22, 23, 24, 25, 26, 27, 28, 29, 30, 31, 32, 33, 34, 35 or 36 nucleotides.

3. An isolated small RNA molecule comprising or consisting of the following sequence:
A) a nucleotide sequence as shown in any one of SEQ ID NO: 1 to SEQ ID NO: 133;
B) a sequence having at least 70%, at least 75%, at least 80%, at least 85%, at least 90%, at least 95%, 96%, 97%, 98%, 99% sequence identity with a nucleotide sequence as shown in any one of SEQ ID NO: 1 to SEQ ID NO: 133;
C) a sequence having up to 10 nucleotide substitutions, deletions, or additions compared with a nucleotide sequence as shown in any one of SEQ ID NO: 1 to SEQ ID NO: 133, preferably a sequence having 9, 8, 7, 6, 5, 4, 3, 2 or 1 nucleotide substitution, deletion, or addition,
D) a nucleotide sequence capable of hybridizing to a nucleotide sequence as shown in any one of SEQ ID NO: 1 to SEQ ID NO: 133 under stringent conditions, or
E) a nucleotide sequence comprising any one of the core sequences listed in Table 4.

4. An isolated small RNA molecule comprising or consisting of the following sequence:
A) a nucleotide sequence as shown in any one of SEQ ID NO: 6, 7, 28 or 109;
B) a sequence having at least 70%, at least 75%, at least 80%, at least 85%, at least 90%, at least 95%, 96%, 97%, 98%, 99% sequence identity with a nucleotide sequence as shown in any one of SEQ ID NO: 6, 7, 28 or 109, and the sequence targets cyclooxygenase-2 and comprises a core sequence GUUCGA; or
C) a nucleotide sequence as shown in SEQ ID NO: 39 or 29;
D) a sequence having at least 70%, at least 75%, at least 80%, at least 85%, at least 90%, at least 95%, 96%, 97%, 98%, 99% sequence identity with a nucleotide sequence as shown in SEQ ID NO: 39 or 29, and the sequence comprises a core sequence UUGUAG; or
E) a nucleotide sequence as shown in SEQ ID NO: 31, 4, 37, 41 or 44;
F) a sequence having at least 70%, at least 75%, at least 80%, at least 85%, at least 90%, at least 95%, 96%, 97%, 98%, 99% sequence identity with a nucleotide sequence as shown in SEQ ID NO: 31, 4, 37, 41 or 44, and the sequence comprises a core sequence UUUGUG.

5. The isolated small RNA molecule according to claim 3 or 4, which is a single-stranded or a double-stranded small RNA molecule.

6. A precursor miRNA, which may be processed within the host into the isolated small RNA molecule according to any one of claims 3-5.

7. A polynucleotide, which may be transcribed by the host to form the precursor miRNA according to claim 6.

8. An expression vector comprising the isolated nucleic acid molecule according to claim 1 or 2, the isolated small RNA molecule according to any one of claims 3-5, the precursor miRNA according to claim 6, and/or the polynucleotide according to claim 7.

9. A host cell transfected with the expression vector according to claim 8.

10. A pharmaceutical composition comprising a therapeutic effective amount of the isolated nucleic acid molecule according to claim 1 or 2, the isolated small RNA molecule according to any one of claims 3-5, the precursor miRNA according to claim 6, the polynucleotide according to claim 7, the expression vector according to claim 8, or the host cell according to claim 9, as well as one or more pharmaceutically acceptable adjuvants, excipients and/or stabilizers, preferably, the pharmaceutical composition is used for administration via oral, intramuscular, intravenous, subcutaneous, percutaneous, intraarterial, intraperitoneal, intrapulmonary, intracerebrospinal, intraarticular, intrasynovial, intrathecal, intraventrical, and/or inhalation routes.

11. The pharmaceutical composition according to claim 10, wherein the content of the isolated nucleic acid molecule, the isolated small RNA molecule, the precursor miRNA, the polynucleotide, the expression vector or the host cell in the pharmaceutical composition is 0.1-1000 µM, preferably 3.0 µM-300 µM.

12. Use of the isolated nucleic acid molecule according to claim 1 or 2, the isolated small RNA molecule according to any one of claims 3-5, the precursor miRNA according to claim 6, the polynucleotide according to claim 7, the expression vector according to claim 8, the host cell according to claim 9, or the pharmaceutical composition according to claim 10 or 11 in the preparation of a medicament for treating cancer.

13. The use according to claim 12, wherein the cancer is selected from the group consisting of lung cancer, preferably squamous cell carcinoma, small cell lung cancer, non-small cell lung cancer; glioma; digestive system tumor, preferably gastric cancer, colon cancer, rectal cancer, liver cancer, pancreatic cancer; kidney cancer; ovarian cancer; uterine cancer; endometrial cancer; prostate cancer; thyroid cancer; neuroblastoma; brain cancer; glioblastoma multiforme; cervical cancer; bladder cancer; breast cancer; head and neck cancer; rhabdomyosarcoma; Ewing's sarcoma; osteosarcoma; soft tissue sarcoma; nasal NK/T-cell lymphoma; myeloma; melanoma; leukemia, preferably acute lymphoblastic leukemia, acute myelogenous leukemia, acute myeloid leukemia, chronic lymphocytic leukemia, chronic myelogenous leukemia and chronic myeloid leukemia.

14. A method for treating cancer in a subject in need, including administering to the subject a therapeutically effective amount of the isolated nucleic acid molecule according to claim 1 or 2, the isolated small RNA molecule according to any one of claims 3-5, the precursor miRNA according to claim 6, the polynucleotide according to claim 7, the expression vector according to claim 8, the host cell according to claim 9, or the pharmaceutical composition according to claim 10 or 11.

15. A method for treating a disease associated with abnormal gene expression, including administering to the subject a therapeutically effective amount of the isolated nucleic acid molecule according to claim 1 or 2, the isolated small RNA molecule according to any one of claims 3-5, the precursor miRNA according to claim 6, the polynucleotide according to claim 7, the expression vector according to claim 8, the host cell according to claim 9, or the pharmaceutical composition according to claim 10 or 11.

16. A method for inhibiting the expression and/or activity of a gene, including administering to a subject a therapeutically effective amount of the isolated nucleic acid molecule according to claim 1 or 2, the isolated small RNA molecule according to any one of claims 3-5, the precursor miRNA according to claim 6, the polynucleotide according to claim 7, the expression vector according to claim 8, the host cell according to claim 9, or the pharmaceutical composition according to claim 10 or 11.
